# EUROPEAN PATENT SPECIFICATION

(11) **EP 2 245 046 B1**
(45) Date of publication and mention of the grant of the patent: **04.09.2019**
(21) Application number: 09703256.9
(22) Date of filing: 22.01.2009
(51) Int. Cl.: C07K 14/00, G01N 33/68, G01N 33/00

(54) **NOVEL CELL LINES EXPRESSING NAV AND METHODS USING THEM**
NEUE NAV-EXPRIMIERENDE ZELLLINIEN UND METHODEN ZU DEREN VERWENDUNG
NOUVELLES LIGNÉES CELLULAIRES EXPRIMANT NAV ET LEURS PROCÉDÉS D'UTILISATION

(30) Priority: 22.01.2008 US 62023 P; 01.02.2008 US 63219 P
(43) Date of publication of application: 03.11.2010
(73) Proprietor: Chromocell Corporation, North Brunswick, NJ 08902 (US)
(72) Inventor: SHEKDAR, Kambiz, New York, New York 10010 (US)
(74) Representative: Vossius & Partner Patentanwälte Rechtsanwälte mbB
(86) International application number: PCT/US2009/000485
(87) International publication number: WO 2009/094218

(56) References cited:
- US-A1- 2007 048 753
- US-B1- 6 692 965
- COX JAMES J ET AL: "An SCN9A channelopathy causes congenital inability to experience pain" NATURE (LONDON), vol. 444, no. 7121, December 2006 (2006-12), pages 894-898, XP009119589 ISSN: 0028-0836
- JOHNSON D ET AL: "Isoform-specific effects of the [beta]2 subunit on voltage-gated sodium channel gating" JOURNAL OF BIOLOGICAL CHEMISTRY 20060908 AMERICAN SOCIETY FOR BIOCHEMISTRY AND MOLECULAR BIOLOGY INC. US, vol. 281, no. 36, 8 September 2006 (2006-09-08), pages 25875-25881, XP009119612
- MEADOWS L S ET AL: "Functional modulation of human brain Nav1.3 sodium channels, expressed in mammalian cells, by auxiliary beta1, beta2 and beta3 subunits" NEUROSCIENCE, vol. 114, no. 3, 11 October 2002 (2002-10-11), pages 745-753, XP009119588 ISSN: 0306-4522
- SEONG-HOON KO ET AL: "Modulation of Nav1.5 by [beta]1 and [beta]3-subunit co-expression in mammalian cells" PFLÜGERS ARCHIV - EUROPEAN JOURNAL OF PHYSIOLOGY, SPRINGER, BERLIN, DE, vol. 449, no. 4, 1 January 2005 (2005-01-01), pages 403-412, XP019343973 ISSN: 1432-2013
- ISOM LORI L ET AL: "Functional Co-expression of the beta-1 and Type IIA alpha Subunits of Sodium Channels in a Mammalian Cell Line" JOURNAL OF BIOLOGICAL CHEMISTRY, vol. 270, no. 7, 1995, pages 3306-3312, XP009119599 ISSN: 0021-9258
- KLUGBAUER N ET AL: "STRUCTURE AND FUNCTIONAL EXPRESSION OF A NEW MEMBER OF THE TETRODOTOXIN-SENSITIVE VOLTAGE-ACTIVATED SODIUM CHANNEL FAMILY FROMHUMAN NEUROENDOCRINE CELLS" EMBO JOURNAL, OXFORD UNIVERSITY PRESS, SURREY, GB, vol. 14, no. 6, 15 March 1995 (1995-03-15), pages 1084-1090, XP002069908 ISSN: 0261-4189
- CATTERALL WILLIAM A ET AL: "International Union of Pharmacology. XXXIX. Compendium of voltage-gated ion channels: sodium channels." PHARMACOLOGICAL REVIEWS DEC 2003, vol. 55, no. 4, December 2003 (2003-12), pages 575-578, XP009119613 ISSN: 0031-6997 cited in the application

## Description

### Background

The voltage-gated sodium ion channel family referred to as the NaV family are large and complex molecules that are expressed in the central nervous system, including the brain, in the peripheral nervous system and in muscle, including cardiac muscle. All of the family members are important clinical targets for managing a variety of conditions including epilepsy, muscle paralysis and pain. NaV channels are cell membrane embedded proteins comprising an alpha subunit and one or more beta subunits. Genes coding for ten alpha subunits and four beta subunits have been identified (see, e.g., Catterall et al., Pharmacol Rev. 55:575-578 (2003); Isom, Neuroscientist, 7:42-54 (2001)). The alpha subunit forms the ion pore and is thought to be responsible for selective sodium conduction and voltage-dependent activation and inactivation (see, e.g., Liu et al., Assay Drug Dev Tech, 4(1):37-48 (2006)). Beta subunits have been shown to modify expression levels and biophysical characteristics of some alpha subunits. Liu et al., *supra.* Both the alpha and beta subunits are differentially expressed in different tissues. *Id.*

The discovery of new and improved therapeutics that specifically target NaV family members has been hampered by the lack of cell-based systems and especially cell-based systems that are amenable to high throughput formats for identifying and testing NaV modulators. Cell-based systems are preferred for drug discovery and validation because they provide a functional assay for a compound as opposed to cell-free systems, which only provide a binding assay. Moreover, cell-based systems have the advantage of simultaneously testing cytotoxicity. The present invention addresses this need.

### Summary of the Invention

We have discovered new and useful cells and cell lines that express functional NaV or subunits thereof. The disclosure thus relates to a plurality (i.e., pool or population) of cells that express a heterologous NaV alpha subunit, a first heterologous NaV beta subunit and a second heterologous NaV beta subunit of a NaV protein, wherein the NaV subunits form a NaV protein (e.g., a native NaV). In some embodiments, the first and second beta subunits are different. In some embodiments, the NaV expression is stable. In some embodiments, the cells are from a cell line (i.e., clonal).

In one aspect of the invention, the invention provides a cell line, or a cell from the cell line, engineered to stably express a heterologous human NaV alpha 9 subunit and two different human NaV beta subunits of a human NaV protein, wherein the human NaV subunits form a human NaV 1.7, wherein the two different human NaV beta subunits are a beta 1 subunit and a beta 2 subunit. In a related aspect, the invention provides a collection of individually isolated cells expressing said NaV subunits.

The disclosure also includes an isolated cell, a plurality of cells, and a collection of individually isolated cells that have been selected based on their expression of the heterologous NaV subunits.

In some embodiments of the invention, the cell is grown or maintained in the absence of antibiotics.

In some embodiments, the cells of this invention express no endogenous NaV subunit.

In the present invention, the NaV protein in the cells is human NaV (e.g., human NaV 1.7). The NaV alpha subunit is an alpha 9subunit. The first and second NaV beta subunits are a beta 1 subunit and a beta 2 subunit.

In a further embodiment, the heterologous NaV alpha 9 subunit comprises the amino acid sequence set forth in SEQ ID NO:27.;

In a further embodiment, the heterologous NaV alpha subunit is encoded by a nucleic acid sequence set forth in SEQ ID NO:13.

In some embodiments, the first and second NaV beta subunits are individually selected from the group consisting of:
a beta 1 subunit having the amino acid sequence set forth in SEQ ID NO:30;
a beta 2 subunit having the amino acid sequence set forth in SEQ ID NO:31;
a polypeptide with at least 95% sequence identity to any one of SEQ ID NOS:30-31, wherein the polypeptide modulates a voltage-gated ion channel;
   a polypeptide encoded by a nucleic acid sequence individually selected from the group consisting of: SEQ ID NOS:16-17; a polypeptide encoded by a nucleic acid that hybridizes under stringent conditions to any one of SEQ ID NOS:16-17; and a polypeptide encoded by a nucleic acid with at least 95% sequence identity to any one of SEQ ID NOS:16-17. The heterologous NaV alpha subunit is a human NaV alpha 9 subunit. The human alpha 9 subunit may comprise (1) the amino acid sequence set forth in SEQ ID NO:27; or (2) an amino acid sequence encoded by a nucleic acid sequence set forth in SEQ ID NO:13. The first heterologous NaV beta subunit may be a human beta 1 subunit. The human beta 1 subunit may comprise (1) the amino acid sequence set forth in SEQ ID NO: 30, or (2) an amino acid sequence encoded by a nucleic acid sequence set forth in SEQ ID NO:16. The second heterologous NaV beta subunit may be a human beta 2 subunit. The human beta 2 subunit may comprise (1) the amino acid sequence set forth in SEQ ID NO:31, or (2) an amino acid sequence encoded by a nucleic acid sequence set forth in SEQ ID NO:17.

In some embodiments, the native NaV may comprise a polypeptide comprising an amino acid sequence set forth in SEQ ID NO:27, a polypeptide comprising the amino acid sequence set forth in SEQ ID NO:30; and a polypeptide comprising the amino acid sequence set forth in SEQ ID NO:31.

In another aspect, the disclosure provides a plurality of cells that express at least one heterologous NaV alpha subunit and at least one heterologous NaV beta subunit, wherein the cells are grown in the absence of selective pressure. In yet another aspect, the disclosure provides an isolated cell expressing at least one heterologous NaV alpha subunit and at least one heterologous NaV beta subunit, wherein the cell is grown in the absence of selective pressure. In a related aspect, the disclosure provides a collection of such isolated cells. In these aspects, the alpha and beta subunits can be, for example, those described above and elsewhere in the specification. Selection pressure is applied in cell culture to select cells with desired sequences or traits, and is usually achieved by linking the expression of a polypeptide of interest with the expression of a selection marker that imparts to the cells resistance to a corresponding selective agent or pressure. Antibiotic selection includes, without limitation, the use of antibiotics (e.g., puromycin, neomycin, G418, hygromycin, bleomycin and the like). Non-antibiotic selection includes, without limitation, the use of nutrient deprivation, exposure to selective temperatures, and exposure to mutagenic conditions where selection marker may be e.g., glutamine synthetase, dihydrofolate reductase (DHFR), oabain, thymidine kinase (TK), hypoxanthine guanine phosphororibosyltransferase (HGPRT). In the instant aspects of the invention, none of such selection steps are applied to the cells in culture.

The invention also provides methods for producing the cell or cell line of the invention. In these methods, one introduces into host cells (e.g., any suitable eukaryotic cells, such as cultured mammalian cells, e.g., 293T cells), a coding sequence for a human NaV alpha 9 subunit, a coding sequence for a first human NaV beta subunit and a coding sequence for a second human NaV beta subunit, said coding sequences being linked operably to transcriptional regulatory sequences such that the coding sequences can be expressed in the host cells, wherein the first and second human NaV beta subunits are different, wherein the first and second human NaV beta subunits are a beta 1 subunit and a beta 2 subunit. Then one can introduce into the host cells a first molecular beacon that detects the expression of the human NaV alpha 9 subunit, a second molecular beacon that detects the expression of the first human NaV beta subunit and a third molecular beacon that detects the expression of the second human NaV beta subunit. One can then isolate cells that stably express the human NaV alpha 9 subunit, the first human NaV beta subunit and the second human NaV beta subunit, and thereby obtain the cell or cell line of the invention. In some embodiments, the cells so made express human NaV, such as NaV 1.7. The molecular beacons may comprise a fluorescent moiety such that fluorescent signal is emitted and detected when the molecular beacons specifically bind to their respective targets. Cell isolation can be performed by a flow cytometer, e.g., a fluorescence activated cell sorter (FACS).

The invention also provides methods for identifying a human NaV 1.7 modulator. The methods comprise contacting the cells of the invention with a test compound; and detecting a change in a human NaV 1.7 function in the cells, wherein a change in said function indicates that the test compound is a human NaV 1.7 modulator. The test compound may be a small molecule, a polypeptide, a peptide, or an antibody or an antigen-binding portion thereof. The test compound may be a library of compounds, such as a small molecule library, a combinatorial library, a peptide library or an antibody library. The detecting step may be, without limitation, a membrane potential assay, electrophysiology assay or a binding assay.

Also encompassed by the disclosure is a method for producing a cell or a collection of clonal cell lines expressing a NaV alpha subunit, a first NaV beta subunit or a second NaV beta subunit of a NaV protein, or any combination thereof by gene activation. According to the method, one or more nucleic acid molecules, for example, a promoter, are inserted upstream of an endogenous NaV alpha subunit, a first NaV beta subunit and/or a second NaV beta subunit, for example by homologous recombination such that the inserted nucleic acid activates expression of the NaV subunit. Methods of gene activation are well-known in the art. In some embodiments, the method further comprises introducing into the host cells a first molecular beacon that detects the expression of the NaV alpha subunit, a second molecular beacon that detects the expression of the first NaV beta subunit and a third molecular beacon that detects the expression of the second NaV beta subunit and isolating cells that express the NaV alpha subunit, the first NaV beta subunit and the second NaV beta subunit, thereby obtaining a cell or a collection of clonal cell lines expressing a NaV alpha subunit, a first NaV beta subunit and a second NaV beta subunit of a NaV protein, wherein the NaV subunits form a native NaV. Also within the invention is a cell or clonal cell line produced by such gene activation method.

### Detailed Disclosure

Unless otherwise defined, all technical and scientific terms used herein have the same meaning as commonly understood by one of ordinary skill in the art to that this invention belongs. Exemplary methods and materials are described below, although methods and materials similar or equivalent to those described herein can also be used in the practice or testing of the present invention. Although a number of documents are cited herein, this citation does not constitute an admission that any of these documents forms part of the common general knowledge in the art. Throughout this specification and claims, the word "comprise," or variations such as "comprises" or "comprising" will be understood to imply the inclusion of a stated integer or group of integers but not the exclusion of any other integer or group of integers. The materials, methods, and examples are illustrative only and not intended to be limiting.

In order that the present invention may be more readily understood, certain terms are first defined. Additional definitions are set forth throughout the specification.

As used herein, the term "native" protein (e.g., ion channel protein) refers to a protein that does not have a heterologous amino acid sequence appended or inserted to it. For example, "native NaV" used herein includes NaV proteins that do not have a tag sequence that is expressed on the polypeptide level. By referring to NaV proteins as native, applicants do not intend to exclude NaV variants that comprise an amino acid substitution, mutation or deletion, or variants that are fragments or spliced forms of naturally occurring, or previously known NaV proteins.

The term "stable" or "stably expressing" refers to the ability of a cell line or cells to express a nucleic acid of interest at consistent levels over a period of time (e.g., one week, two weeks, four weeks, one month, two months, six months, or longer).

The term "isolated cell" refers to a cell that is separated from other cells in culture. For example, when a well of a multi-well tissue culture plate contains only one cell, that cell is considered an isolated cell.

The term "cell line" or "clonal cell line" refers to a population of cells that are all progeny of a single original cell. As used herein, cell lines are maintained *in vitro* in cell culture and may be frozen in aliquots to establish a bank of clonal cells.

The term "stringent conditions" or "stringent hybridization conditions" describes temperature and salt conditions for hybridizing one or more nucleic acid probes to a nucleic acid sample and washing off probes that have not bound specifically to target nucleic acids in the sample. Stringent conditions are known to those skilled in the art and can be found in Current Protocols in Molecular Biology, John Wiley & Sons, N.Y. (1989), 6.3.1-6.3.6. Aqueous and nonaqueous methods are described in that reference and either can be used. An example of stringent hybridization conditions is hybridization in 6X SSC at about 45°C, followed by at least one wash in 0.2X SSC, 0.1% SDS at 65°C. High stringent conditions include hybridization in 0.5M sodium phosphate, 7% SDS at 65°C, followed by at least one wash at 0.2X SSC, 1% SDS at 65°C.

The phrase "percent identical" or "percent identity" in connection with amino acid and/or nucleic acid sequences refers to the similarity between at least two different sequences. This percent identity can be determined by standard alignment algorithms, for example, the Basic Local Alignment Tool (BLAST) described by Altshul et al. ((1990) J. Mol. Biol., 215: 403-410); the algorithm of Needleman et al. ((1970) J. Mol. Biol., 48: 444-453); or the algorithm of Meyers et al. ((1988) Comput. Appl. Biosci., 4: 11-17). A set of parameters may be the Blosum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller ((1989) CABIOS, 4:11-17) that has been incorporated into the ALIGN program (version 2.0), using a PAM120 weight residue table, a gap length penalty of 12 and a gap penalty of 4. The percent identity is usually calculated by comparing sequences of similar length. Protein analysis software matches similar sequences using measures of similarity assigned to various substitutions, deletions and other modifications, including conservative amino acid substitutions. For instance, GCG contains programs such as "Gap" and "Bestfit" that can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA using default or recommended parameters, a program in GCG Version 6.1. FASTA (e.g., FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)). The length of polypeptide sequences compared for homology will generally be at least about 16 amino acid residues, usually at least about 20 residues, more usually at least about 24 residues, typically at least about 28 residues, and preferably more than about 35 residues.

The phrase "substantially as set out," "substantially identical" or "substantially homologous" means that the relevant amino acid or nucleotide sequence will be identical to or have insubstantial differences (through conserved amino acid substitutions) in comparison to the sequences that are set out. Insubstantial differences include minor amino acid changes, such as 1 or 2 substitutions in a 50 amino acid sequence of a specified region.

A NaV "modulator" refers to a compound that alters a biological activity of a NaV, e.g., ion conductance via a NaV. A NaV modulator may act upon all or upon a specific subset of NaVs or NaV subunits. Modulators include, but are not limited to, agonists (potentiators or activators) and antagonists (inhibitors or blockers). A Nav agonist refers to a compound that increases a biological activity of a Nav. A NaV antagonist refers to a compound that decreases a biological activity of a Nav.

A "functional NaV" refers to a NaV that has one or more of the biological activities of a naturally occurring or endogenously expressed NaV. Biological activities of NaV include, but are not limited to, voltage-gated sodium conductance, and can be assessed via pharmacological responses such as inhibition by lidocaine and tetrodotoxin (TTX). Other compounds that are pharmacologically active on NaV and can thus be used to assess the functionality of an introduced NaV include sodium channel openers - compounds that hold the channel in its open state, for example, veratridine, and various scorpion and other venoms.

A "heterologous" or "introduced" NaV subunit means that the NaV subunit is encoded by a polynucleotide introduced into a host cell, or by an endogenous NaV-coding sequence whose expression is activated (e.g., by gene activation technology) by externally introduced factors such as transcriptional regulatory elements. A "heterologous NaV" refers to NaV comprising one or more heterologous NaV subunits.

In a first aspect, the invention provides cells (e.g., isolated cells, clonal cells, or mixtures of clonal cells) and cell lines that express (e.g., stably) one or more heterologous (introduced) NaV subunits (e.g., native NaV subunits). The cells and cell lines constitutively express the NaV subunits. The cells and cell lines may be modulated by channel openers such as veratridine and scorpion venom, or membrane voltage changes. The cells or cell lines may express two, three, or more heterologous NaV subunits (an alpha subunit and two types of beta subunits). In related embodiments, the cells or cell lines express a functional heterologous NaV.

The NaV is human NaV 1.7.

In the present disclosure, the NaV alpha subunit may be any NaV alpha subunit, including any of the human NaV alpha subunits. Accordingly, in some embodiments, the cells of the disclosure may comprise a nucleic acid that encodes a NaV alpha 1 (SCN1A) (SEQ ID NO: 20); a NaV alpha 2 (SCN2A) (SEQ ID NO: 21); a NaV alpha 3 (SCN3A) (SEQ ID NO: 22); a NaV alpha 4 (SCN4A) (SEQ ID NO: 23); a NaV alpha 5 (SCN5A) (SEQ ID NO: 24); a NaV alpha 7 (SCN7A) (SEQ ID NO: 25) (∀6 and ∀7 subunits are synonymous); a NaV alpha 8 (SCN8A) (SEQ ID NO: 26); a NaV alpha 9 (SCN9A) (SEQ ID NO: 27); a NaV alpha 10 (SCN10A) (SEQ ID NO: 28); or a NaV alpha 11 (SCN11A) (SEQ ID NO: 29). In some embodiments the NaV alpha subunit coding nucleic acid is selected from the group consisting of SEQ ID NOS: 6-15.

Any one of the NaV alpha subunits may be co-introduced, or sequentially introduced, and co-expressed with any one or more NaV beta subunits to generate the cells of the invention. In some embodiments, the cells stably expresses human NaV beta subunits, for example, a human NaV beta 1 subunit (SCN1B) (SEQ ID NO: 30), a human NaV beta 2 subunit (SCN2B) (SEQ ID NO: 31), a human NaV beta 3 subunit (SCN3B) (SEQ ID NO: 32) and a human NaV beta 4 subunit (SCN4B) (SEQ ID NO: 33). In some embodiments, the NaV beta subunit is encoded by a nucleic acid selected from the group consisting of SEQ ID NOS: 16-19. In some embodiments, the cells are triply transfected with nucleic acids encoding, and expresses, a human NaV alpha 9/SCN9A subunit, a human NaV betal/SCNIB subunit and a human NaV beta 2/SCN2B subunit.

In some embodiments, the present cells and cell lines express an introduced alpha subunit, selected from any one of alpha 1-11, and an introduced beta subunit, selected from any one of beta 1-4, with each combination indicated by a "+" in the following table:

| | Beta 1 | Beta 2 | Beta 3 | Beta 4 |
|---|---|---|---|---|
| Alpha 1 | + | + | + | + |
| Alpha 2 | + | + | + | + |
| Alpha 3 | + | + | + | + |
| Alpha 4 | + | + | + | + |
| Alpha 5 | + | + | + | + |
| Alpha 7 | + | + | + | + |
| Alpha 8 | + | + | + | + |
| Alpha 9 | + | + | + | + |
| Alpha 10 | + | + | + | + |
| Alpha 11 | + | + | + | + |

These cells and cells lines can further express one or more introduced beta subunits independently selected from any one of beta 1-4. In some embodiments, the cells and cell lines of the invention express a NaV channel containing a combination of alpha and beta subunits as shown in the above table, and in further embodiments, the NaV channel in these cell lines further comprise one or more beta subunits selected from any one of beta 1-4.

The nucleic acid encoding the NaV subunit can be genomic DNA or cDNA. In some embodiments, the nucleic acid encoding the NaV subunit comprises one or more substitutions, insertions, or deletions that may or may not result in an amino acid substitution. NaV subunits with modifications within the scope of the invention retain at least one biological property, e.g., its ability to function as, or modulate, a voltage-gated sodium channel or to respond to ion channel openers such as veratridine and scorpion and other venoms and channel blockers such as lidocaine and tetrodotoxin (TTX). Accordingly, nucleic acid sequences substantially identical (e.g., at least about 85% sequence identity) or homologous (e.g., at least about 85% sequence homology) to the sequences disclosed herein are also encompassed by this invention. In some embodiment, the sequence identity can be about 85%, 90%, 95%, 96%, 97%, 98%, 99%, or higher. Alternatively, substantial identity or homology exists when the nucleic acid segments will hybridize under stringent hybridization conditions (e.g., highly stringent hybridization conditions) to the complement of the reference sequence.

In some embodiments, where the nucleotide mutation involves an amino acid substitution, the native amino acid may be replaced by a conservative or non-conservative substitution. In some embodiments, the sequence identity between the original and modified polypeptide sequences can be at least 85%, 90%, 95%, 96%, 97%, 98%, 99% or higher. Those of skill in the art will understand that a conservative amino acid substitution is one in which the amino acid side chains are similar in structure and/or chemical properties and the substitution should not substantially change the structural characteristics of the wild type sequence. In embodiments using a nucleic acid comprising a mutation, the mutation may be a random mutation or a site-specific mutation.

Conservative modifications will produce NaV receptors having functional and chemical characteristics similar to those of the unmodified NaV receptor. A "conservative amino acid substitution" is one in which an amino acid residue is substituted by another amino acid residue having a side chain R group) with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. See e.g. Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

In some embodiments, the NaV subunit-coding nucleic acid sequence further comprises an epitope tag. Such tags may encode, for example, yellow fluorescent protein (YFP), green fluorescent protein (GFP), 6x-HIS (SEQ ID NO: 35), myc, FLAG, or hemagglutinin (HA), S-tag, thioredoxin, autofluorescent proteins, GST, V5, TAP, CBP, BCCP, Maltose binding protein-tag, Nus-tag, Softag 1, Softag 3, Strep-tag, or a variant of the aforementioned. A tag may be used as a marker to determine the expression levels, intracellular localization, protein-protein interaction, regulation, and function of a NaV or a subunit thereof. A tag also may be used to facilitate protein purification and fractionation. These and other tag sequences are known to one of skill in the art and typically correspond to amino acid sequences that may be incorporated into expressed protein products and often selected based on the availability of robust antibodies or protein detection reagents that may be used to report their presence. However, tag sequences described herein are not meant to refer solely to sequences that may be used to modify, at the amino acid level, protein products encoded by the RNAs that are tagged, or to aid in the subsequent detection of any such modified protein products through use of the corresponding antibody or protein detection reagents. See, for example, discussions below in regard to using RNA tags used as "molecular beacons."

Host cells used to produce a cell line of the invention may express one or more endogenous NaV proteins or lack expression of one or more of any NaV protein. The host cell may be a primary, germ, or stem cell, including an embryonic stem cell. The host cell may also be an immortalized cell. The host cell may be derived from a primary or immortalized cell from mesoderm, ectoderm, or endoderm layers. The host cell may be endothelial, epidermal, mesenchymal, neural, renal, hepatic, hematopoietic, or immune cells. For example, the host cells may be intestinal crypt or villi cells, clara cells, colon cells, intestinal cells, goblet cells, enterochromafin cells, enteroendocrine cells. The host cells may be eukaryotic, prokaryotic, mammalian, human, primate, bovine, porcine, feline, rodent, marsupial, murine or other cells. The host cells may also be nonmammalian, such as yeast, insect, fungus, plant, lower eukaryotes and prokaryotes. Such host cells may provide backgrounds that are more divergent for testing with a greater likelihood for the absence of expression products provided by the cell that may interact with the target. In preferred embodiments, the host cell is a mammalian cell. Examples of host cells that may be used for a cell line of the invention include but are not limited to: Chinese hamster ovary (CHO) cells, established neuronal cell lines, pheochromocytomas, neuroblastomas fibroblasts, rhabdomyosarcomas, dorsal root ganglion cells, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573), PC12 (ATCC CRL-1721), HEK293T (ATCC CRL-11268), RBL (ATCC CRL-1378), SH-SY5Y (ATCC CRL-2266), MDCK (ATCC CCL-34), SJ-RH30 (ATCC CRL-2061), HepG2 (ATCC HB-8065), ND7/23 (ECACC 92090903), CHO (ECACC 85050302), Vero (ATCC CCL 81), Caco-2 (ATCC HTB 37), K562 (ATCC CCL 243), Jurkat (ATCC TIB-152), Per.C6 (Crucell, Leiden, The Netherlands), Huvec (ATCC Human Primary PCS 100-010, Mouse CRL 2514, CRL 2515, CRL 2516), HuH-7D12 (ECACC 01042712), 293 (ATCC CRL 10852), A549 (ATCC CCL 185), IMR-90 (ATCC CCL 186), MCF-7 (ATC HTB-22), U-2 OS (ATCC HTB-96), T84 (ATCC CCL 248), or any established cell line (polarized or nonpolarized) or any cell line available from repositories such as The American Type Culture Collection (ATCC, 10801 University Blvd. Manassas, Va. 20110-2209 USA) or European Collection of Cell Cultures (ECACC, Salisbury Wiltshire SP4 0JG England). One of ordinary skill in the art will understand that different known or unknown accessory factors that may interact with or alter the function or expression of the target depending on the choice of host cell type.

The host cell may be a non-human embryonic stem cell that is then used as the basis for the generation of non-human transgenic animals. Non-human embryonic stem cells stably expressing at least one NaV subunit, and preferably a functional heterologous NaV receptor, may be implanted into non-human organisms directly, or their nuclei may be transferred into other non-human cells and these may then be implanted *in vivo* for studying growth and development.

As will be appreciated by those of skill in the art, any vector that is suitable for use with the host cell may be used to introduce a nucleic acid encoding a NaV alpha or beta subunit into the host cell. The vectors comprising the alpha and each of the beta subunits may be the same type or may be of different types. Examples of vectors that may be used to introduce the NaV subunit-encoding nucleic acids into host cells include but are not limited to plasmids, viruses, including retroviruses and lentiviruses, cosmids, artificial chromosomes and may include for example, pCMV-Script, pcDNA3.1 Hygro, pcDNA3.1neo, pcDNA3.1puro, pSV2neo, pIRES puro, pSV2 zeo. In some embodiments, the vectors comprise expression control sequences such as constitutive or conditional promoters. One of ordinary skill in the art will be able to select such sequences. For example, suitable promoters include but are not limited to CMV, TK, SV40 and EF-1α. In some embodiments, the promoters are inducible, temperature regulated, tissue specific, repressible, heat-shock, developmental, cell lineage specific, prokaryotic and/or eukaryotic expressible or temporal promoters or a combination or recombination of unmodified or mutagenized, randomized, shuffled sequences of any one or more of the above. Nucleic acids encoding NaV subunits are preferably constitutively expressed.

In some embodiments, the vector lacks a selectable marker or drug resistance gene. In other embodiments, the vectors optionally comprises a nucleic acid encoding a selectable marker such as a protein that confers drug or antibiotic resistance. Each vector for a sequence encoding a different NaV subunit may have the same or a different drug resistance or other selectable marker. If more than one of the drug resistance markers are the same, simultaneous selection may be achieved by increasing the level of the drug. Suitable markers will be well-known to those of skill in the art and include but are not limited to genes conferring resistance to any one of the following: Neomycin/G418, Puromycin, January 22, 2009, Zeocin, methotrexate and blasticidin. Although drug selection (or selection using any other suitable selection marker) is not a required step, it may be used, if desired, to enrich the transfected cell population for stably transfected cells, provided that the transfected constructs are designed to confer drug resistance. If selection is accomplished using signaling probes, selection performed too soon following transfection can result in some positive cells that may only be transiently and not stably transfected. However, this can be minimized by allowing sufficient cell passage, allowing for dilution of transiently transfected cells, stably integrated cells that do not express the introduced DNA, or cells that generate RNA that may not be efficiently detected by the signaling probes.

In the invention, cells and cell lines of the invention stably express NaV or a NaV subunit. To identify stable expression, a cell line's expression of each NaV subunit is measured over a time course and the expression levels are compared. Stable cell lines will continue expressing the NaV subunits throughout the time course at substantially the same level (e.g., no more than 40%, 30%, 20%, 15%, 10%, 5%, or 2% variation). In some aspects of the invention, the time course may be for at least one week, two weeks, three weeks, or four weeks; or at least one, two, three, four, five, six, seven, eight, or nine months, or at least any length of time in between. Isolated cells can be further characterized, such as by qRT-PCR and single end-point RT-PCR to determine the absolute and/or relative amounts of each NaV subunit being expressed, or by any other conventional method of protein expression analysis.

Also according to the invention, cells and cell lines that express a recombinant form of a NaV hetero-multimer can be characterized for NaV functions, e.g., sodium ion conductance. Suitable assays include, without limitation, a membrane potential assay using sodium or potassium as a NaV activator, an electrophysiology assay, and a binding or panning assay.

The present cells and cell lines can be used in a collection, each set of cells or each cell line expressing one form of NaV, e.g., NaV comprised of various combinations (e.g., dimers, trimers, etc.) of alpha and beta subunits or variants (e.g., mutants, fragments, or spliced variants) of the subunits, or a multimer of only an alpha or beta subunit. The collection may include, for example, cell lines expressing two or more of the aforementioned NaV receptors.

To make cells and cell lines of the invention, one can use, for example, the technology described in U.S. Patent 6,692,965 and WO/2005/079462.This technology provides real-time assessment of millions of cells such that any desired number of clones (from hundreds to thousands of clones). Using cell sorting techniques, such as flow cytometric cell sorting (e.g., with a FACS machine) or magnetic cell sorting (e.g., with a MACS machine), one cell per well is automatically deposited with high statistical confidence in a culture vessel (such as a 96 well culture plate). The speed and automation of the technology allows multigene recombinant cell lines to be readily isolated.

Using the technology, the RNA sequence for each NaV subunit may be detected using a signaling probe, also referred to as a molecular beacon or fluorogenic probe. In some embodiments, the vector containing the NaV subunit-coding sequence has an additional sequence coding for an RNA tag sequence. "Tag sequence" refers to a nucleic acid sequence that is an expressed RNA or portion of an RNA that is to be detected by a signaling probe. Signaling probes may detect a variety of RNA sequences, any of which may be used as tags, including those encoding peptide and protein tags described above. Signaling probes may be directed against the tag by designing the probes to include a portion that is complementary to the sequence of the tag. The tag sequence may be a 3' untranslated region of the plasmid that is cotranscribed with a NaV transcript and comprises a target sequence for signaling probe binding. The tag sequence can be in frame with the protein-coding portion of the message of the gene or out of frame with it, depending on whether one wishes to tag the protein produced. Thus, the tag sequence does not have to be translated for detection by the signaling probe. The tag sequences may comprise multiple target sequences that are the same or different, wherein one signaling probe hybridizes to each target sequence. The tag sequence may be located within the RNA encoding the gene of interest, or the tag sequence may be located within a 5'- or 3'-untranslated region. The tag sequences may be an RNA having secondary structure. The structure may be a three-arm junction structure. In some embodiments, the signaling probe detects a sequence within the NaV subunit-coding sequence.

Nucleic acids comprising a sequence encoding a NaV subunit, optionally a sequence coding for a tag sequence, and optionally a nucleic acid encoding a selectable marker may be introduced into selected host cells by well known methods. The methods include but not limited to transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, FUGENE 6, FUGENE HD, TFX-10, TFX-20, TFX-50, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, AND METAFECTINE.

Following transfection of the DNA constructs into cells and subsequent drug selection (if used), or following gene activation as described above, molecular beacons (e.g., fluorogenic probes), each of which is targeted to a different tag sequence and differentially labeled, may be introduced into the cells, and a flow cytometric cell sorter is used to isolate cells positive for their signals (multiple rounds of sorting may be carried out). In one embodiment, the flow cytometric cell sorter is a FACS machine. MACS can also be used. Signal-positive cells have taken up and may have integrated into their genomes at least one copy of the introduced NaV sequence(s). Cells introduced with one or more of the NaV subunits are identified. By way of example, the NaV subunit sequences may be integrated at different locations of the genome in the cell. The expression level of the introduced genes encoding the NaV subunits may vary based upon copy number or integration site. Further, cells comprising one or more of the NaV subunits may be obtained wherein one or more of the introduced genes encoding a NaV subunit is episomal or results from gene activation.

Signaling probes useful in this invention are known in the art and generally are oligonucleotides comprising a sequence complementary to a target sequence and a signal emitting system so arranged that no signal is emitted when the probe is not bound to the target sequence and a signal is emitted when the probe binds to the target sequence. By way of non-limiting illustration, the signaling probe may comprise a fluorophore and a quencher positioned in the probe so that the quencher and fluorophore are brought together in the unbound probe. Upon binding between the probe and the target sequence, the quencher and fluorophore separate, resulting in emission of signal. International publication WO/2005/079462, for example, describes a number of signaling probes that may be used in the production of the present cells and cell lines. The methods described above for introducing nucleic acids into cells may be used to introduce signaling probes.

Where tag sequences are used, the vector for each of the NaV subunit can comprise the same or a different tag sequence. Whether the tag sequences are the same or different, the signaling probes may comprise different signal emitters, such as different colored fluorophores and the like so that expression of each subunit may be separately detected. By way of illustration, the signaling probe that specifically detects NaV alpha subunit mRNA can comprise a red fluorophore, the probe that detects the first NaV beta subunit can comprise a green fluorophore, and the probe that detects the second NaV beta subunit can comprise a blue fluorophore. Those of skill in the art will be aware of other means for differentially detecting the expression of the three subunits with a signaling probe in a triply transfected cell.

In one embodiment, the signaling probes are designed to be complementary to either a portion of the RNA encoding a NaV subunit or to portions of their 5' or 3' untranslated regions. Even if the signaling probe designed to recognize a messenger RNA of interest is able to detect spuriously endogenously expressed target sequences, the proportion of these in comparison to the proportion of the sequence of interest produced by transfected cells is such that the sorter is able to discriminate the two cell types.

The expression level of an introduced NaV subunit may vary from cell line to cell line. The expression level in a cell line also may decrease over time due to epigenetic events such as DNA methylation and gene silencing and loss of transgene copies. These variations can be attributed to a variety of factors, for example, the copy number of the transgene taken up by the cell, the site of genomic integration of the transgene, and the integrity of the transgene following genomic integration. One may use FACS to evaluate expression levels. Cells expressing an introduced NaV subunit at desired levels can be isolated by, e.g., FACS. Signaling probes also may be re-applied to previously generated cells or cell lines, for example, to determine if and to what extent the cells are still positive for any one or more of the RNAs for which they were originally isolated.

Once cells expressing all three NaV subunits are isolated, they may be cultured for a length of time sufficient to identify those stably expressing all the desired subunits. In one embodiment, isolated cells may be grown individually or pooled to give rise to populations of cells. Individual or multiple cell lines may also be grown separately or pooled. If a pool of cell lines is producing a desired activity, it can be further fractionated until the cell line or set of cell lines having this effect is identified. This may make it easier to maintain large numbers of cell lines without the requirements for maintaining each separately.

The ease to isolate and re-isolate from a mixed cell population those cells expressing desired RNAs at appropriate levels makes it possible to maintain cell lines under no or minimal drug selection pressure. Thus, in some preferred embodiments, cells and cell lines of the invention are maintained in culture without any selective drug. In further embodiments, cells and cell lines are maintained without any antibiotics. As used herein, cell maintenance refers to culturing cells after they have been selected for their NaV expression through, e.g., cell sorting. Maintenance does not refer to the optional step of growing cells in a selective drug (e.g., an antibiotic) prior to cell sorting where drug resistance marker(s) introduced into the cells allow enrichment of stable transfectants in a mixed population.

Drug-free cell maintenance provides a number of advantages. For examples, drug-resistant cells do not always express the co-transfected transgene of interest at adequate levels, because the selection relies on survival of the cells that have taken up the drug resistant gene, with or without the transgene. Further, selective drugs are often mutagenic or otherwise interferes the physiology of the cells, leading to skewed results in cell-based assays. For example, selective drugs may decrease susceptibility to apoptosis (Robinson et al., Biochemistry, 36(37):11169-11178 (1997)), increase DNA repair and drug metabolism (Deffie et al., Cancer Res. 48(13):3595-3602 (1988)), increase cellular pH (Thiebaut et al., J Histochem Cytochem. 38(5):685-690 (1990); Roepe et al., Biochemistry. 32(41):11042-11056 (1993); Simon et al., Proc Natl Acad Sci U S A. 91(3):1128-1132 (1994)), decrease lysosomal and endosomal pH (Schindler et al., Biochemistry. 35(9):2811-2817 (1996); Altan et al., J Exp Med. 187(10):1583-1598 (1998)), decrease plasma membrane potential (Roepe et al., Biochemistry. 32(41):11042-11056 (1993)), increase plasma membrane conductance to chloride (Gill et al., Cell. 71(1):23-32 (1992)) and ATP (Abraham et al., Proc Natl Acad Sci U S A. 90(1):312-316 (1993)), and increase rates of vesicle transport (Altan et al., Proc Natl Acad Sci U S A. 96(8):4432-4437 (1999)). Thus, the cells and cell lines of this invention allow screening assays that are free from any artifact caused by selective drugs. In some preferred embodiments, cells are not cultured with selective drugs such as antibiotics before or after cell sorting so that cells with desired properties are isolated by sorting even without beginning with an enriched cell population.

In another aspect, the invention provides methods of using the cells and cell lines of the invention. The cells and cell lines of the invention may be used in any application for which a functional NaV subunit(s) or complete NaV ion channel is needed. The cells and cell lines may be used, for example, in an *in vitro* cell-based assay or an *in vivo* assay where the cells are implanted in an animal (e.g., a non-human mammal) to, e.g., screen for NaV modulators; produce protein for crystallography and binding studies; and investigate compound selectivity and dosing, receptor/compound binding kinetic and stability, and effects of receptor expression on cellular physiology (e.g., electrophysiology, protein trafficking, protein folding, and protein regulation). The present cells and cell lines also can be used in knock down studies to study the roles of specific NaV subunits.

Cell lines expressing various combinations of alpha and beta subunits (e.g., naturally occurring heterotrimers or nonnaturally occurring heterotrimers) can be used separately or together as a collection to identify NaV modulators, including those specific for a particular NaV, a particular subunit of a NaV, or a particular combination of NaV subunits, and to obtain information about the activities of individual subunits. The invention also provides methods for using modulators specific for particular modified forms; such information may be useful in determining whether NaV has naturally occurring modified forms. Using the present cell and cell lines can help determine whether different forms of NaV are implicated in different NaV pathologies and allow selection of disease- or tissue-specific NaV modulators for highly targeted treatment of NaV-related pathologies.

As used herein, a "modulator" includes any substance or compound that has modulating activity with respect to at least one NaV subunit. The modulator can be a NaV agonist (potentiator or activator) or antagonist (inhibitor or blocker), including partial agonists or antagonists, selective agonists or antagonists and inverse agonists, and can be an allosteric modulator. A substance or compound is a modulator even if its modulating activity changes under different conditions or concentrations. In some aspects of the invention, the modulator alters the selectivity of an ion channel. For example, a modulator may affect what ions are able to pass through an ion channel.

To identify a NaV modulator, one can expose a cell line of the invention to a test compound under conditions in which the NaV would be expected to be functional and detect a statistically significant change (e.g., p < 0.05) in NaV activity compared to a suitable control, e.g., cells from the cell line that are not contacted with the test compound. Alternatively, or in addition positive and/or negative controls using known agonists or antagonists, cells expressing different combinations of NaV subunits may be used. In some embodiments, the NaV activity to be detected and/or measured is membrane depolarization, change in membrane potential, or fluorescence resulting from such membrane changes.

In some embodiments, one or more cell lines of the invention are exposed to a plurality of test compounds, for example, a library of test compounds. A library of test compounds can be screened using the cell lines of the invention to identify one or more modulators. The test compounds can be chemical moieties including small molecules, polypeptides, peptides, peptide mimetics, antibodies or antigen-binding portions thereof. In the case of antibodies, they may be non-human antibodies, chimeric antibodies, humanized antibodies, or fully human antibodies. The antibodies may be intact antibodies comprising a full complement of heavy and light chains or antigen-binding portions, including antibody fragments (such as Fab and Fab, Fab', F(ab')₂, Fd, Fv, dAb and the like), single subunit antibodies (scFv), single domain antibodies, all or an antigen-binding portion of a heavy or light chain.

In some embodiments, prior to exposure to a test compound, the cells may be modified by pretreatment with, for example, enzymes, including mammalian or other animal enzymes, plant enzymes, bacterial enzymes, protein modifying enzymes and lipid modifying enzymes. Such enzymes can include, for example, kinases, proteases, phosphatases, glycosidases, oxidoreductases, transferases, hydrolases, lyases, isomerases, ligases and the like. For example, in some embodiments, cells are pretreated with at least one proteolytic enzyme such as trypsin or furin. Alternatively, the cells may be exposed to the test compound first followed by treatment to identify compounds that alter the modification of the NaV by the treatment.

In some embodiments, large compound collections are tested for NaV-modulating activity in a cell-based, functional, high-throughput screen (HTS), e.g., using 96, 384, 1536, or higher density well format. Hits from the HTS screen may be subsequently tested in additional assays to confirm function, e.g., determination of their chemical structures, testing of structurally related compounds to optimize activity and specificity, and further testing in animal models. In some embodiments, the therapeutic potential of modulators is tested in animal models to assess their usefulness in the treatment of human diseases and conditions, including but not limited to epilepsy, periodic paralysis, cardiac diseases, CNS diseases, ataxia, and pain (chronic or acute), loss of ability to feel pain. By way of example, a human NaV 1.7 expressing cell line of the invention can be used to identify a NaV 1.7 antagonist for use as an analgesic to reduce or eliminate pain.

These and other embodiments of the invention may be further illustrated in the following non-limiting Examples.

### EXAMPLES

### Example 1 Generating Expression Constructs

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and neomycin/kanamycin resistance cassettes.

### Example 2 Generating a Stable NaV1.7 Heterotrimer-Expressing Cell Line

We transfected 293T cells with three separate plasmids, each encoding one NaV subunit. Although drug selection is optional, we included one drug resistance marker. The NaV sequences were the human SCN9A, SCN1B and SCN2B under the control of CMV, SV40 and TK promoters in frame. An untranslated sequence encoding a tag for detection by a signaling probe is also present along with a sequence encoding a drug resistance marker. The cells were selected in media containing appropriate levels of drug for 10-14 days. After selection, the cells were expanded in media lacking drug in number sufficient for expansion and clone isolation.

### A. Clone isolation procedure:

Cells were harvested at the start of the experiment and maintained in cell culture media. The signaling probes were delivered to the cells by lipid transfection. The cells were then dissociated and collected for analysis and sorted using a fluorescence activated cell sorter. Standard analytical methods were used to gate cells fluorescing above background and to isolate cells falling within the defined gate. Positive cells were sorted as single cells into 96 well plates.

### B. Target Sequences detected by signaling probes

The following tag sequences were used for the NaV 1.7 subunit transgenes.
**Target 1**
   5'-GTTCTTAAGGCACAGGAACTGGGAC-3' (SEQ ID NO: 1)
**Target 2**
   5'-GAAGTTAACCCTGTCGTTCTGCGAC-3' (SEQ ID NO: 2)
**Target 3**
   5'-GTTCTATAGGGTCTGCTTGTCGCTC-3' (SEQ ID NO: 3)

### C. Signaling probes

Signaling probes used to detect the tag sequences were supplied as 100µM stocks. Although other probes could be used, the sequences of the probes used in this case were:
**Signaling probe 1** - This probe binds target 1. 
5' - Cy5 GCCAGTCCCAGTTCCTGTGCCTTAAGAACCTCGC
BHQ2 quench -3' (SEQ ID NO: 4)
**Signaling probe 2** - This probe binds target 2 
5'- Cy5.5 GCGAGTCGCAGAACGACAGGGTTAACTTCCTCGC
BHQ2 quench -3' (SEQ ID NO: 5)
**Signaling probe 3** - This probe binds target 3 
5'- Fam GCGAGAGCGACAAGCAGACCCTATAGAACCTCGC
BHQ1 quench -3' (SEQ ID NO: 34)
 BHQ2 in Signaling probes 1 and 2 can be replaced by BHQ1 or gold particle. BHQ1 in Signaling probe 3 can be replaced by BHQ2, gold particle, or DABCYL.

### Example 3 Characterizing Cell LineS for Native NaV Function

This example illustrates protocols for characterizing cell lines expressing native NaV 1.7.

NaV 1.7-expressing cell lines are maintained under standard cell culture conditions in Dulbecco's Modified Eagles medium supplemented with 10% fetal bovine serum, glutamine and HEPES. On the day before assay, the cells are harvested from stock plates using cell dissociation buffer and plated into black clear-bottom 384 well assay plates. The assay plates are maintained in a 37°C cell culture incubator under 5% CO₂ / 95% O₂ for 22-24 hours. The media is then removed from the assay plates and blue membrane potential dye (Molecular Devices Inc) is added. The cells are incubated with blue membrane potential dye for an hour at 37°C. To test compounds in the assay, test compounds and drugs prepared in assay buffer are added to the cell plate in a Hamamatsu FDSS fluorescent plate reader (the first addition). In a second addition step, veratidine and scorpion venom are added. The response of the cells is monitored over time. (Note that as control, the first addition can include buffer only, with no test compound added.) The fluorescent plate reader measures cell fluorescence in images taken of the cell plate once per second and displays the data as relative florescence units. Baseline values are collected before the instrument transfers 20 µl of the test compound onto the cells. Images are then collected for an additional 4 minutes. The activity of the compound is determined by measuring the change in fluorescence it produces in the NaV-expressing cells as compared with the fluorescence produced in control cells.

To compare the activity of cells expressing all three NaV 1.7 subunits to control cells, one can conduct the above-described assay on both types of cells without a test compound (buffer only). One also can run the assay using cells expressing all three NaV 1.7 subunits and cells expressing only the alpha subunit treated with either H-89 (N-[2-(p-Bromocinnamylamino)ethyl]-5-isoquinolinesulfonamide dihydrobromide; designated C-18) or citalopram hydrobromide (designated K21) as test compounds.

### LISTING OF SEQUENCES

Target 1
   5'-GTTCTTAAGGCACAGGAACTGGGAC-3' (SEQ ID NO: 1)
Target 2
   5'-GAAGTTAACCCTGTCGTTCTGCGAC-3' (SEQ ID NO: 2)
Target 3
   5'-GTTCTATAGGGTCTGCTTGTCGCTC-3' (SEQ ID NO: 3)
Signaling probe 1 (binds target 1)
   **5' - Cy5** GCCAGTCCCAGTTCCTGTGCCTTAAGAACCTCGC **BHQ2 quench -3'** (SEQ ID NO: 4)
Signaling probe 2 - (binds target 2)
   **5'- Cy5.5** GCGAGTCGCAGAACGACAGGGTTAACTTCCTCGC **BHQ2 quench** - **3'** (SEQ ID NO: 5)
Homo sapiens (H.s.) SCN1A 
atggagcaaacagtgcttgtaccaccaggacctgacagcttcaacttcttcaccagagaatctcttgcggctattgaaagac
gcattgcagaagaaaaggcaaagaatcccaaaccagacaaaaaagatgacgacgaaaatggcccaaagccaaatagtg
acttggaagctggaaagaaccttccatttatttatggagacattcctccagagatggtgtcagagcccctggaggacctgga
cccctactatatcaataagaaaacttttatagtattgaataaagggaaggccatcttccggttcagtgccacctctgccctgtac
attttaactcccttcaatcctcttaggaaaatagctattaagattttggtacattcattattcagcatgctaattatgtgcactattttg
acaaactgtgtgtttatgacaatgagtaaccctcctgattggacaaagaatgtagaatacaccttcacaggaatatatacttttg
aatcacttataaaaattattgcaaggggattctgtttagaagattttactttccttcgggatccatggaactggctcgatttcactg
tcattacatttgcgtacgtcacagagtttgtggacctgggcaatgtctcggcattgagaacattcagagttctccgagcattga
agacgatttcagtcattccaggcctgaaaaccattgtgggagccctgatccagtctgtgaagaagctctcagatgtaatgatc
ctgactgtgttctgtctgagcgtatttgctctaattgggctgcagctgttcatgggcaacctgaggaataaatgtatacaatggc
ctcccaccaatgcttccttggaggaacatagtatagaaaagaatataactgtgaattataatggtacacttataaatgaaactgt
ctttgagtttgactggaagtcatatattcaagattcaagatatcattatttcctggagggtttttagatgcactactatgtggaaat
agctctgatgcaggccaatgtccagagggatatatgtgtgtgaaagctggtagaaatcccaattatggctacacaagctttga
taccttcagttgggctttttgtccttgtttcgactaatgactcaggacttctgggaaaatctttatcaactgacattacgtgctgct
gggaaaacgtacatgatatttttgtattggtcattttcttgggctcattctacctaataaatttgatcctggctgtggtggccatgg
cctacgaggaacagaatcaggccaccttggaagaagcagaacagaaagaggccgaatttcagcagatgattgaacagct
taaaaagcaacaggaggcagctcagcaggcagcaacggcaactgcctcagaacattccagagagcccagtgcagcag
gcaggctctcagacagctcatctgaagcctctaagttgagttccaagagtgctaaggaaagaagaaatcggaggaagaaa
agaaaacagaaagagcagtctggtggggaagagaaagatgaggatgaattccaaaaatctgaatctgaggacagcatca
 
ggaggaaaggttttcgcttctccattgaagggaaccgattgacatatgaaaagaggtactcctccccacaccagtctttgttg
agcatccgtggctccctattttcaccaaggcgaaatagcagaacaagccttttcagctttagaggcgagcaaaggatgtg
ggatctgagaacgacttcgcagatgatgagcacagcacctttgaggataacgagagccgtagagattccttgtttgtgcccc
gacgacacggagagagacgcaacagcaacctgagtcagaccagtaggtcatcccggatgctggcagtgtttccagcgaa
tgggaagatgcacagcactgtggattgcaatggtgtggtttccttggttggtggaccttcagttcctacatcgcctgttggaca
gcttctgccagagggaacaaccactgaaactgaaatgagaaagagaaggtcaagttctttccacgtttccatggactttctag
aagatccttcccaaaggcaacgagcaatgagtatagccagcattctaacaaatacagtagaagaacttgaagaatccaggc
agaaatgcccaccctgttggtataaattttccaacatattcttaatctgggactgttctccatattggttaaaagtgaaacatgttg
tcaacctggttgtgatggacccatttgttgacctggccatcaccatctgtattgtcttaaatactcttttcatggccatggagcact
atccaatgacggaccatttcaataatgtgcttacagtaggaaacttggttttcactgggatctttacagcagaaatgtttctgaa
aattattgccatggatccttactattatttccaagaaggctggaatatctttgacggttttattgtgacgcttagcctggtagaactt
ggactcgccaatgtggaaggattatctgttctccgttcatttcgattgctgcgagttttcaagttggcaaaatcttggccaacgtt
aaatatgctaataaagatcatcggcaattccgtgggggctctgggaaatttaaccctcgtcttggccatcatcgtcttcatttttg
ccgtggtcggcatgcagctctttggtaaaagctacaaagattgtgtctgcaagatcgccagtgattgtcaactcccacgctgg
cacatgaatgacttcttccactccttcctgattgtgttccgcgtgctgtgtggggagtggatagagaccatgtgggactgtatg
gaggttgctggtcaagccatgtgccttactgtcttcatgatggtcatggtgattggaaacctagtggtcctgaatctctttctgg
ccttgcttctgagctcatttagtgcagacaaccttgcagccactgatgatgataatgaaatgaataatctccaaattgctgtgga
taggatgcacaaaggagtagcttatgtgaaaagaaaaatatatgaatttattcaacagtccttcattaggaaacaaaagattta
gatgaaattaaaccacttgatgatctaaacaacaagaaagacagttgtatgtccaatcatacagcagaaattgggaaagatct
tgactatcttaaagatgtaaatggaactacaagtggtataggaactggcagcagtgttgaaaaatacattattgatgaaagtga
ttacatgtcattcataaacaaccccagtcttactgtgactgtaccaattgctgtaggagaatctgactttgaaaatttaaacacg
gaagactttagtagtgaatcggatctggaagaaagcaaagagaaactgaatgaaagcagtagctcatcagaaggtagcac
tgtggacatcggcgcacctgtagaagaacagcccgtagtggaacctgaagaaactcttgaaccagaagcttgtttcactga
aggctgtgtacaaagattcaagtgttgtcaaatcaatgtggaagaaggcagaggaaaacaatggtggaacctgagaagga
cgtgtttccgaatagttgaacataactggtttgagaccttcattgttttcatgattctccttagtagtggtgctctggcatttgaaga
tatatatattgatcagcgaaagacgattaagacgatgttggaatatgctgacaaggttttcacttacattttcattctggaaatgct
tctaaaatgggtggcatatggctatcaaacatatttcaccaatgcctggtgttggctggacttcttaattgttgatgtttcattggt
cagtttaacagcaaatgccttgggttactcagaacttggagccatcaaatctctcaggacactaagagctctgagacctctaa
gagccttatctcgatttgaagggatgagggtggttgtgaatgcccttttaggagcaattccatccatcatgaatgtgcttctggt
ttgtcttatattctggctaatttcagcatcatgggcgtaaatttgtttgctggcaaattctaccactgtattaacaccacaactggt
gacaggtttgacatcgaagacgtgaataatcatactgattgcctaaaactaatagaaagaaatgagactgctcgatggaaaa
atgtgaaagtaaactttgataatgtaggatttgggtatctctctttgcttcaagttgccacattcaaaggatggatggatataatgt
atgcagcagttgattccagaaatgtggaactccagcctaagtatgaagaaagtctgtacatgtatctttactttgttattttcatca
tctttgggtccttcttcaccttgaacctgtttattggtgtcatcatagataatttcaaccagcagaaaaagaagtttggaggtcaa
gacatctttatgacagaagaacagaagaaatactataatgcaatgaaaaaattaggatcgaaaaaaccgcaaaagcctata
cctcgaccaggaaacaaatttcaaggaatggtctttgacttcgtaaccagacaagtttttgacataagcatcatgattctcatct
gtcttaacatggtcacaatgatggtggaaacagatgaccagagtgaatatgtgactaccattttgtcacgcatcaatctggtgt
tcattgtgctatttactggagagtgtgtactgaaactcatctctctacgccattattattttaccattggatggaatatttttgatttg
tggttgtcattctctccattgtaggtatgtttcttgccgagctgatagaaaagtatttcgtgtccccctaccctgttccgagtgatcc
gtcttgctaggattggccgaatcctacgtctgatcaaaggagcaaaggggatccgcacgctgctctttgctttgatgatgtcc
cttcctgcgttgtttaacatcggcctcctactcttcctagtcatgttcatctacgccatctttgggatgtccaactttgcctatgtta
agagggaagttgggatcgatgacatgttcaactttgagacctttggcaacagcatgatctgcctattccaaattacaacctctg
ctggctgggatggattgctagcacccattctcaacagtaagccacccgactgtgaccctaataaagttaaccctggaagctc
agttaagggagactgtgggaacccatctgttggaatttttcttttttgtcagttacatcatcatatccttcctggttgtggtgaacat
gtacatcgcggtcatcctggagaacttcagtgttgctactgaagaaagtgcagagcctctgagtgaggatgactttgagatgt
tctatgaggtttgggagaagtttgatcccgatgcaactcagttcatggaatttgaaaaattatctcagtttgcagctgcgcttga
accgcctctcaatctgccacaaccaaacaaactccagctcattgccatggatttgcccatggtgagtggtgaccggatccac
tgtcttgatatcttatttgcttttacaaagcgggttctaggagagagtggagagatggatgctctacgaatacagatggaagag
cgattcatggcttccaatccttccaaggtctcctatcagccaatcactactactttaaaacgaaaacaagaggaagtatctgct
gtcattattcagcgtgcttacagacgccaccttttaaagcgaactgtaaaacaagcttcctttacgtacaataaaaacaaaatc
 
aaaggtggggctaatcttcttataaaagaagacatgataattgacagaataaatgaaaactctattacagaaaaaactgatct
gaccatgtccactgcagcttgtccaccttcctatgaccgggtgacaaagccaattgtggaaaaacatgagcaagaaggcaa
agatgaaaaagccaaagggaaataa (SEQ ID NO: 6)
H.s. SCN2A 
atggcacagtcagtgctggtaccgccaggacctgacagcttccgcttctttaccagggaatcccttgctgctattgaacaac
gcattgcagaagagaaagctaagagacccaaacaggaacgcaaggatgaggatgatgaaaatggcccaaagccaaac
agtgacttggaagcaggaaaatctcttccatttatttatggagacattcctccagagatggtgtcagtgcccctggaggatctg
gacccctactatatcaataagaaaacgtttatagtattgaataaagggaaagcaatctctcgattcagtgccacccctgccctt
tacattttaactcccttcaaccctattagaaaattagctattaagattttggtacattctttattcaatatgctcattatgtgcacgatt
cttaccaactgtgtatttatgaccatgagtaaccctccagactggacaaagaatgtggagtatacctttacaggaatttatacttt
tgaatcacttattaaaatacttgcaaggggcttttgttagaagattcacatttttacgggatccatggaattggttggatttcaca
gtcattacttttgcatatgtgacagagtttgtggacctgggcaatgtctcagcgttgagaacattcagagttctccgagcattga
aaacaatttcagtcattccaggcctgaagaccattgtgggggccctgatccagtcagtgaagaagctttctgatgtcatgatct
tgactgtgttctgtctaagcgtgtttgcgctaataggattgcagttgttcatgggcaacctacgaaataaatgtttgcaatggcct
ccagataattcttcctttgaaataaatatcacttccttctttaacaattcattggatgggaatggtactactttcaataggacagtg
agcatatttaactgggatgaatatattgaggataaaagtcacttttattttttagaggggcaaaatgatgctctgctttgtggcaa
cagctcagatgcaggccagtgtcctgaaggatacatctgtgtgaaggctggtagaaaccccaactatggctacacgagctt
tgacacctttagttgggcctttttgtccttatttcgtctcatgactcaagacttctgggaaaacctttatcaactgacactacgtgct
gctgggaaaacgtacatgatatttttgtgctggtcattttcttgggctcattctatctaataaatttgatcttggctgtggtggcca
tggcctatgaggaacagaatcaggccacattggaagaggctgaacagaaggaagctgaatttcagcagatgctcgaaca
gttgaaaaagcaacaagaagaagctcaggcggcagctgcagccgcatctgctgaatcaagagacttcagtggtgctggtg
ggataggagtttttcagagagttcttcagtagcatctaagttgagctccaaaagtgaaaaagagctgaaaaacagaagaaa
gaaaaagaaacagaaagaacagtctggagaagaagagaaaaatgacagagtccgaaaatcggaatctgaagacagcat
aagaagaaaaggtttccgtttttccttggaaggaagtaggctgacatatgaaaagagattttcttctccacaccagtccttactg
agcatccgtggctcccttttctctccaagacgcaacagtagggcgagccttttcagcttcagaggtcgagcaaaggacattg
gctctgagaatgactttgctgatgatgagcacagcacctttgaggacaatgacagccgaagagactctctgttcgtgccgca
cagacatggagaacggcgccacagcaatgtcagccaggccagccgtgcctccagggtgctccccatcctgcccatgaat
gggaagatgcatagcgctgtggactgcaatggtgtggtctccctggtcgggggcccttctaccctcacatctgctgggcag
ctcctaccagagggcacaactactgaaacagaaataagaaagagacggtccagttcttatcatgtttccatggatttattgga
agatcctacatcaaggcaaagagcaatgagtatagccagtattttgaccaacaccatggaagaacttgaagaatccagaca
gaaatgcccaccatgctggtataaatttgctaatatgtgtttgatttgggactgttgtaaaccatggttaaaggtgaaacacctt
gtcaacctggttgtaatggacccatttgttgacctggccatcaccatctgcattgtcttaaatacactcttcatggctatggagc
actatcccatgacggagcagttcagcagtgtactgtctgttggaaacctggtcttcacagggatcttcacagcagaaatgtttc
tcaagataattgccatggatccatattattactttcaagaaggctggaatatttttgatggttttattgtgagccttagtttaatgga
acttggtttggcaaatgtggaaggattgtcagttctccgatcattccggctgctccgagttttcaagttggcaaaatcttggcca
actctaaatatgctaattaagatcattggcaattctgtgggggctctaggaaacctcaccttggtattggccatcatcgtcttcat
ttttgctgtggtcggcatgcagctctttggtaagagctacaaagaatgtgtctgcaagatttccaatgattgtgaactcccacgc
tggcacatgcatgactttttccactccttcctgatcgtgttccgcgtgctgtgtggagagtggatagagaccatgtgggactgt
atggaggtcgctggccaaaccatgtgccttactgtcttcatgatggtcatggtgattggaaatctagtggttctgaacctcttctt
ggccttgcttttgagttccttcagttctgacaatcttgctgccactgatgatgataacgaaatgaataatctccagattgctgtgg
gaaggatgcagaaaggaatcgatttgttaaaagaaaaatacgtgaatttattcagaaagcctttgttaggaagcagaaagct
ttagatgaaattaaaccgcttgaagatctaaataataaaaagacagctgtatttccaaccataccaccatagaaataggcaa
agacctcaattatctcaaagacggaaatggaactactagtggcataggcagcagtgtagaaaaatatgtcgtggatgaaagt
gattacatgtcatttataaacaaccctagcctcactgtgacagtaccaattgctgttggagaatctgactttgaaaatttaaatac
tgaagaattcagcagcgagtcagatatggaggaaagcaaagagaagctaaatgcaactagttcatctgaaggcagcacg
gttgatattggagctcccgccgagggagaacagcctgaggttgaacctgaggaatcccttgaacctgaagcctgttttacag
aagactgtgtacggaagttcaagtgttgtcagataagcatagaagaaggcaaagggaaactctggtggaatttgaggaaaa
 
catgctataagatagtggagcacaattggttcgaaaccttcattgtcttcatgattctgctgagcagtggggctctggcctttga
agatatatacattgagcagcgaaaaaccattaagaccatgttagaatatgctgacaaggttttcacttacatattcattctggaa
atgctgctaaagtgggttgcatatggttttcaagtgtattttaccaatgcctggtgctggctagacttcctgattgttgatgtctca
ctggttagcttaactgcaaatgccttgggttactcagaacttggtgccatcaaatccctcagaacactaagagctctgaggcc
actgagagctttgtcccggtttgaaggaatgagggttgttgtaaatgctcttttaggagccattccatctatcatgaatgtacttc
tggtttgtctgatcttttggctaatattcagtatcatgggagtgaatctctttgctggcaagttttaccattgtattaattacaccact
ggagagatgtttgatgtaagcgtggtcaacaactacagtgagtgcaaagctctcattgagagcaatcaaactgccaggtgg
aaaaatgtgaaagtaaactttgataacgtaggacttggatatctgtctctacttcaagtagccacgtttaagggatggatggat
attatgtatgcagctgttgattcacgaaatgtagaattacaacccaagtatgaagacaacctgtacatgtatctttattttgtcatc
tttattatttttggttcattctttaccttgaatcttttcattggtgtcatcatagataacttcaaccaacagaaaaagaagtttggaggt
caagacatttttatgacagaagaacagaagaaatactacaatgcaatgaaaaaactgggttcaaagaaaccacaaaaaccc
atacctcgacctgctaacaaattccaaggaatggtctttgattttgtaaccaaacaagtctttgatatcagcatcatgatcctcat
ctgccttaacatggtcaccatgatggtggaaaccgatgaccagagtcaagaaatgacaaacattctgtactggattaatctgg
tgtttattgttctgttcactggagaatgtgtgctgaaactgatctctcttcgttactactatttcactattggatggaatatttttgattt
tgtggtggtcattctctccattgtaggaatgtttctggctgaactgatagaaaagtattttgtgtcccctaccctgttccgagtgat
ccgtcttgccaggattggccgaatcctacgtctgatcaaaggagcaaaggggatccgcacgctgctctttgctttgatgatgt
cccttcctgcgttgtttaacatcggcctccttcttttcctggtcatgttcatctacgccatctttgggatgtccaattttgcctatgtt
aagagggaagttgggatcgatgacatgttcaactttgagacctttggcaacagcatgatctgcctgttccaaattacaacctct
gctggctgggatggattgctagcacctattcttaatagtggacctccagactgtgaccctgacaaagatcaccctggaagct
cagttaaaggagactgtgggaacccatctgttgggatttctttttgtcagttacatcatcatatccttcctggttgtggtgaaca
tgtacatcgcggtcatcctggagaacttcagtgttgctactgaagaaagtgcagagcctctgagtgaggatgactttgagatg
ttctatgaggtttgggagaagtttgatcccgatgcgacccagtttatagagtttgccaaactttctgattttgcagatgccctgga
tcctcctcttctcatagcaaaacccaacaaagtccagctcattgccatggatctgcccatggtgagtggtgaccggatccact
gtcttgacatcttatttgcttttacaaagcgtgttttgggtgagagtggagagatggatgcccttcgaatacagatggaagagc
gattcatggcatcaaacccctccaaagtctcttatgagcccattacgaccacgttgaaacgcaaacaagaggaggtgtctgc
tattattatccagagggcttacagacgctacctcttgaagcaaaaagttaaaaaggtatcaagtatatacaagaaagacaaag
gcaaagaatgtgatggaacacccatcaaagaagatactctcattgataaactgaatgagaattcaactccagagaaaaccg
atatgacgccttccaccacgtctccaccctcgtatgatagtgtgaccaaaccagaaaaagaaaaatttgaaaaagacaaatc
agaaaaggaagacaaagggaaagatatcagggaaagtaaaaagtaa (SEQ ID NO: 7)
H.s. SCN3A 
atggcacaggcactgttggtacccccaggacctgaaagcttccgcctttttactagagaatctcttgctgctatcgaaaaacg
tgctgcagaagagaaagccaagaagcccaaaaaggaacaagataatgatgatgagaacaaaccaaagccaaatagtga
cttggaagctggaaagaaccttccatttatttatggagacattcctccagagatggtgtcagagcccctggaggacctggatc
cctactatatcaataagaaaacttttatagtaatgaataaaggaaaggcaattttccgattcagtgccacctctgccttgtatattt
taactccactaaaccctgttaggaaaattgctatcaagattttggtacattctttattcagcatgcttatcatgtgcactattttgac
caactgtgtatttatgaccttgagcaaccctcctgactggacaaagaatgtagagtacacattcactggaatctatacctttga
gtcacttataaaaatcttggcaagagggttttgcttagaagatttacgtttcttcgtgatccatggaactggctggatttcagtgt
cattgtgatggcatatgtgacagagtttgtggacctgggcaatgtctcagcgttgagaacattcagagttctccgagcactga
aaacaatttcagtcattccaggtttaaagaccattgtgggggccctgatccagtcggtaaagaagctttctgatgtgatgatcc
tgactgtgttctgtctgagcgtgtttgctctcattgggctgcagctgttcatgggcaatctgaggaataaatgtttgcagtggcc
cccaagcgattctgcttttgaaaccaacaccacttcctactttaatggcacaatggattcaaatgggacatttgttaatgtaaca
atgagcacatttaactggaaggattacattggagatgacagtcacttttatgttttggatgggcaaaaagaccctttactctgtg
gaaatggctcagatgcaggccagtgtccagaaggatacatctgtgtgaaggctggtcgaaaccccaactatggctacaca
agctttgacacctttagctgggctttcctgtctctatttcgactcatgactcaagactactgggaaaatctttaccagttgacatta
cgtgctgctgggaaaacatacatgatatttttttgtcctggtcattttcttgggctcattttatttggtgaatttgatcctggctgtggt
ggccatggcctatgaggagcagaatcaggccaccttggaagaagcagaacaaaaagaggccgaatttcagcagatgctc
gaacagcttaaaaagcaacaggaagaagctcaggcagttgcggcagcatcagctgcttcaagagatttcagtggaatagg
 
tgggttaggagagctgttggaaagttcttcagaagcatcaaagttgagttccaaaagtgctaaagaatggaggaaccgaag
gaagaaaagaagacagagagagcaccttgaaggaaacaacaaaggagagagagacagctttcccaaatccgaatctga
agacagcgtcaaaagaagcagcttccttttctccatggatggaaacagactgaccagtgacaaaaaattctgctcccctcat
cagtctctcttgagtatccgtggctccctgttttccccaagacgcaatagcaaaacaagcattttcagtttcagaggtcgggca
aaggatgttggatctgaaaatgactttgctgatgatgaacacagcacatttgaagacagcgaaagcaggagagactcactg
tttgtgccgcacagacatggagagcgacgcaacagtaacgttagtcaggccagtatgtcatccaggatggtgccagggctt
ccagcaaatgggaagatgcacagcactgtggattgcaatggtgtggtttccttggtgggtggaccttcagctctaacgtcac
ctactggacaacttcccccagagggcaccaccacagaaacggaagtcagaaagagaaggttaagctcttaccagatttca
atggagatgctggaggattcctctggaaggcaaagagccgtgagcatagccagcattctgaccaacacaatggaagaact
tgaagaatctagacagaaatgtccgccatgctggtatagatttgccaatgtgttcttgatctgggactgctgtgatgcatggtta
aaagtaaaacatcttgtgaatttaattgttatggatccatttgttgatcttgccatcactatttgcattgtcttaaataccctctttatg
gccatggagcactaccccatgactgagcaattcagtagtgtgttgactgtaggaaacctggtctttactgggattttcacagc
agaaatggttctcaagatcattgccatggatccttattactatttccaagaaggctggaatatctttgatggaattattgtcagcct
cagtttaatggagcttggtctgtcaaatgtggagggattgtctgtactgcgatcattcagactgcttagagttttcaagttggca
aaatcctggcccacactaaatatgctaattaagatcattggcaattctgtgggggctctaggaaacctcaccttggtgttggcc
atcatcgtcttcatttttgctgtggtcggcatgcagctctttggtaagagctacaaagaatgtgtctgcaagatcaatgatgact
gtacgctcccacggtggcacatgaacgacttcttccactccttcctgattgtgttccgcgtgctgtgtggagagtggatagag
accatgtgggactgtatggaggtcgctggccaaaccatgtgccttattgttttcatgttggtcatggtcattggaaaccttgtgg
ttctgaacctctttctggccttattgttgagttcatttagctcagacaaccttgctgctactgatgatgacaatgaaatgaataatct
gcagattgcagtaggaagaatgcaaaagggaattgattatgtgaaaaataagatgcgggagtgtttccaaaaagccttttta
gaaagccaaaagttatagaaatccatgaaggcaataagatagacagctgcatgtccaataatactggaattgaaataagca
aagagcttaattatcttagagatgggaatggaaccaccagtggtgtaggtactggaagcagtgttgaaaaatacgtaatcga
tgaaaatgattatatgtcattcataaacaaccccagcctcaccgtcacagtgccaattgctgttggagagtctgactttgaaaa
cttaaatactgaagagttcagcagtgagtcagaactagaagaaagcaaagagaaattaaatgcaaccagctcatctgaagg
aagcacagttgatgttgttctaccccgagaaggtgaacaagctgaaactgaacccgaagaagaccttaaaccggaagcttg
ttttactgaaggatgtattaaaaagtttccattctgtcaagtaagtacagaagaaggcaaagggaagatctggtggaatcttcg
aaaaacctgctacagtattgttgagcacaactggtttgagactttcattgtgttcatgatccttctcagtagtggtgcattggcctt
tgaagatatatacattgaacagcgaaagactatcaaaaccatgctagaatatgctgacaaagtctttacctatatattcattctg
gaaatgcttctcaaatgggttgcttatggatttcaaacatatttcactaatgcctggtgctggctagatttcttgatcgttgatgttt
ctttggttagcctggtagccaatgctcttggctactcagaactcggtgccatcaaatcattacggacattaagagctttaagac
ctctaagagccttatcccggtttgaaggcatgagggtggttgtgaatgctcttgttggagcaattccctctatcatgaatgtgct
gttggtctgtctcatcttctggttgatctttagcatcatgggtgtgaatttgtttgctggcaagttctaccactgtgttaacatgaca
acgggtaacatgtttgacattagtgatgttaacaatttgagtgactgtcaggctcttggcaagcaagctcggtggaaaaacgt
gaaagtaaactttgataatgttggcgctggctatcttgcactgcttcaagtggccacatttaaaggctggatggatattatgtat
gcagctgttgattcacgagatgttaaacttcagcctgtatatgaagaaaatctgtacatgtatttatactttgtcatctttatcatctt
tgggtcattcttcactctgaatctattcattggtgtcatcatagataacttcaaccagcagaaaaagaagtttggaggtcaagac
atctttatgacagaggaacagaaaaaatattacaatgcaatgaagaaacttggatccaagaaacctcagaaacccatacctc
gcccagcaaacaaattccaaggaatggtctttgattttgtaaccagacaagtctttgatatcagcatcatgatcctcatctgcct
caacatggtcaccatgatggtggaaacggatgaccagggcaaatacatgaccctagttttgtcccggatcaacctagtgttc
attgttctgttcactggagaatttgtgctgaagctcgtctccctcagacactactacttcactataggctggaacatctttgacttt
gtggtggtgattctctccattgtaggtatgtttctggctgagatgatagaaaagtattttgtgtcccctaccttgttccgagtgatc
cgtcttgccaggattggccgaatcctacgtctgatcaaaggagcaaaggggatccgcacgctgctctttgctttgatgatgtc
ccttcctgcgttgtttaacatcggcctcctgctcttcctggtcatgtttatctatgccatctttgggatgtccaactttgcctatgtta
aaaaggaagctggaattgatgacatgttcaactttgagacctttggcaacagcatgatctgcttgttccaaattacaacctctg
ctggctgggatggattgctagcacctattcttaatagtgcaccacccgactgtgaccctgacacaattcaccctggcagctca
gttaagggagactgtgggaacccatctgttgggatttctttttgtcagttacatcatcatatccttcctggttgtggtgaacatg
tacatcgcggtcatcctggagaacttcagtgttgctactgaagaaagtgcagagcccctgagtgaggatgactttgagatgtt
ctatgaggtttgggaaaagtttgatcccgatgcgacccagtttatagagttctctaaactctctgattttgcagctgccctggat
cctcctcttctcatagcaaaacccaacaaagtccagcttattgccatggatctgcccatggtcagtggtgaccggatccactg
tcttgatattttatttgcctttacaaagcgtgttttgggtgagagtggagagatggatgcccttcgaatacagatggaagacag
 
gtttatggcatcaaacccctccaaagtctcttatgagcctattacaaccactttgaaacgtaaacaagaggaggtgtctgccg
ctatcattcagcgtaatttcagatgttatcttttaaagcaaaggttaaaaaatatatcaagtaactataacaaagaggcaattaaa
gggaggattgacttacctataaaacaagacatgattattgacaaactaaatgggaactccactccagaaaaaacagatggg
agttcctctaccacctctcctccttcctatgatagtgtaacaaaaccagacaaggaaaagtttgagaaagacaaaccagaaa
aagaaagcaaaggaaaagaggtcagagaaaatcaaaagtaa (SEQ ID NO: 8)
H.s. SCN4A 
atggccagaccatctctgtgcaccctggtgcctctgggccctgagtgcttgcgccccttcacccgggagtcactggcagcc
atagaacagcgggcggtggaggaggaggcccggctgcagcggaataagcagatggagattgaggagcccgaacgga
agccacgaagtgacttggaggctggcaagaacctacccatgatctacggagacccccgccggaggtcatcggcatccc
cctggaggacctggatccctactacagcaataagaagaccttcatcgtactcaacaagggcaaggccatcttccgcttctcc
gccacacctgctctctacctgctgagccccttcagcgtagtcaggcgcggggccatcaaggtgctcatccatgcgctgttca
gcatgttcatcatgatcaccatcttgaccaactgcgtattcatgaccatgagtgacccgcctccctggtccaagaatgtggag
tacaccttcacaggcatctacacctttgagtccctcatcaagatactggcccgaggcttctgtgtcgacgacttcacattcctc
cgggacccctggaactggctggacttcagtgtcatcatgatggcgtacctgacagagtttgtggacttgggcaacatctcag
ccctgaggaccttccgggtgctgcgggccctcaaaaccatcacggtcatcccagggctgaagacgatcgtgggggccct
gatccagtcggtgaaaaagctgtcggatgtgatgatcctcactgtcttctgcctgagcgtctttgcgctggtaggactgcagc
tcttcatgggaaacctgaggcagaagtgtgtgcgctggcccccgccgttcaacgacaccaacaccacgtggtacagcaat
gacacgtggtacggcaatgacacatggtatggcaatgagatgtggtacggcaatgactcatggtatgccaacgacacgtg
gaacagccatgcaagctgggccaccaacgatacctttgattgggacgcctacatcagtgatgaagggaacttctacttcctg
gagggctccaacgatgccctgctctgtgggaacagcagtgatgctgggcactgccctgagggttatgagtgcatcaagac
cgggcggaaccccaactatggctacaccagctatgacaccttcagctgggccttcttggctctcttccgcctcatgacacag
gactattgggagaacctcttccagctgacccttcgagcagctggcaagacctacatgatcttcttcgtggtcatcatcttcctg
ggctctttctacctcatcaatctgatcctggccgtggtggccatggcatatgccgagcagaatgaggccaccctggccgag
gataaggagaaagaggaggagtttcagcagatgcttgagaagttcaaaaagcaccaggaggagctggagaaggccaag
gccgcccaagctctggaaggtggggaggcagatggggacccagcccatggcaaagactgcaatggcagcctggacac
atcgcaaggggagaagggagccccgaggcagagcagcagcggagacagcggcatctccgacgccatggaagaactg
gaagaggcccaccaaaagtgcccaccatggtggtacaagtgcgcccacaaagtgctcatatggaactgctgcgccccgt
ggctgaagttcaagaacatcatccacctgatcgtcatggacccgttcgtggacctgggcatcaccatctgcatcgtgctcaa
caccctcttcatggccatggaacattaccccatgacggagcactttgacaacgtgctcactgtgggcaacctggtcttcaca
ggcatcttcacagcagagatggttctgaagctgattgccatggacccctacgagtatttccagcagggttggaatatcttcga
cagcatcatcgtcaccctcagcctggtagagctaggcctggccaacgtacagggactgtctgtgctacgctccttccgtctg
ctgcgggtcttcaagctggccaagtcgtggccaacgctgaacatgctcatcaagatcattggcaattcagtgggggcgctg
ggtaacctgacgctggtgctggctatcatcgtgttcatcttcgccgtggtgggcatgcagctgtttggcaagagctacaagg
agtgcgtgtgcaagattgccttggactgcaacctgccgcgctggcacatgcatgatttcttccactccttcctcatcgtcttcc
gcatcctgtgcggggagtggatcgagaccatgtgggactgcatggaggtggccggccaagccatgtgcctcaccgtcttc
ctcatggtcatggtcatcggcaatcttgtggtcctgaacctgttcctggctctgctgctgagctccttcagcgccgacagtctg
gcagcctcggatgaggatggcgagatgaacaacctgcagattgccatcgggcgcatcaagttgggcatcggctttgccaa
ggccttcctcctggggctgctgcatggcaagatcctgagccccaaggacatcatgctcagcctcggggaggctgacggg
gccggggaggctggagaggcgggggagactgcccccgaggatgagaagaaggagccgcccgaggaggacctgaa
gaaggacaatcacatcctgaaccacatgggcctggctgacggccccccatccagcctcgagctggaccaccttaacttcat
caacaaccccctacctgaccatacaggtgcccatcgcctccgaggagtccgacctggagatgcccaccgaggaggaaac
cgacactttctcagagcctgaggatagcaagaagccgccgcagcctctctatgatgggaactcgtccgtctgcagcacag
ctgactacaagcccccgaggaggaccctgaggagcaggcagaggagaaccccgaggggagcagcctgaggagtg
cttcactgaggcctgcgtgcagcgctggccctgcctctacgtggacatctcccagggccgtgggaagaagtggtggactc
tgcgcagggcctgcttcaagattgtcgagcacaactggttcgagaccttcattgtcttcatgatcctgctcagcagtggggct
ctggccttcgaggacatctacattgagcagcggcgagtcattcgcaccatcctagaatatgccgacaaggtcttcacctaca
tcttcatcatggagatgctgctcaaatgggtggcctacggctttaaggtgtacttcaccaacgcctggtgctggctcgacttcc
 
tcatcgtggatgtctccatcatcagcttggtggccaactggctgggctactcggagctgggacccatcaaatccctgcggac
actgcgggccctgcgtcccctgagggcactgtcccgattcgagggcatgagggtggtggtgaacgccctcctaggcgcc
atcccctccatcatgaatgtgctgcttgtctgcctcatcttctggctgatcttcagcatcatgggtgtcaacctgtttgccggcaa
gttctactactgcatcaacaccaccacctctgagaggttcgacatctccgaggtcaacaacaagtctgagtgcgagagcctc
atgcacacaggccaggtccgctggctcaatgtcaaggtcaactacgacaacgtgggtctgggctacctctccctcctgcag
gtggccaccttcaagggttggatggacatcatgtatgcagccgtggactcccgggagaaggaggagcagccgcagtacg
aggtgaacctctacatgtacctctactttgtcatcttcatcatctttggctccttcttcaccctcaacctcttcattggcgtcatcatt
gacaacttcaaccagcagaagaagaagttagggggaaagacatctttatgacggaggaacagaagaaatactataacg
ccatgaagaagcttggctccaagaagcctcagaagccaattccccggccccagaacaagatccagggcatggtgtatga
cctcgtgacgaagcaggccttcgacatcaccatcatgatcctcatctgcctcaacatggtcaccatgatggtggagacagac
aaccagagccagctcaaggtggacatcctgtacaacatcaacatgatcttcatcatcatcttcacaggggagtgcgtgctca
agatgctcgccctgcgccagtactacttcaccgttggctggaacatctttgacttcgtggtcgtcatcctgtccattgtgggcct
tgccctctctgacctgatccagaagtacttcgtgtcacccacgctgttccgtgtgatccgcctggcgcggattgggcgtgtcc
tgcggctgatccgcggggccaagggcatccggacgctgctgttcgccctcatgatgtcgctgcctgccctcttcaacatcg
gcctcctcctcttcctggtcatgttcatctactccatcttcggcatgtccaactttgcctacgtcaagaaggagtcgggcatcga
tgatatgttcaacttcgagaccttcggcaacagcatcatctgcctgttcgagatcaccacgtcggccggctgggacgggctc
ctcaaccccatcctcaacagcgggcccccagactgtgaccccaacctggagaacccgggcaccagtgtcaagggtgact
gcggcaacccctccatcggcatctgcttcttctgcagctatatcatcatctccttcctcatcgtggtcaacatgtacatcgccat
catcctggagaacttcaatgtggccacagaggagagcagcgagccccttggtgaagatgactttgagatgttctacgagac
atgggagaagttcgaccccgacgccacccagttcatcgcctacagccgcctctcagacttcgtggacaccctgcaggaac
cgctgaggattgccaagcccaacaagatcaagctcatcacactggacttgcccatggtgccaggggacaagatccactgc
ctggacatcctctttgccctgaccaaagaggtcctgggtgactctggggaaatggacgccctcaagcagaccatggagga
gaagttcatggcagccaacccctccaaggtgtcctacgagcccatcaccaccaccctcaagaggaagcacgaggaggtg
tgcgccatcaagatccagagggcctaccgccggcacctgctacagcgctccatgaagcaggcatcctacatgtaccgcca
cagccacgacggcagcggggatgacgcccctgagaaggaggggctgcttgccaacaccatgagcaagatgtatggcc
acgagaatgggaacagcagctcgccaagcccggaggagaagggcgaggcaggggacgccggacccactatggggc
tgatgcccatcagcccctcagacactgcctggcctcccgcccctcccccagggcagactgtgcgcccaggtgtcaagga
gtctcttgtctag (SEQ ID NO: 9)
H.s. SCN5A 
atggcaaacttcctattacctcggggcaccagcagcttccgcaggttcacacgggagtccctggcagccatcgagaagcg
catggcagagaagcaagcccgcggctcaaccaccttgcaggagagccgagaggggctgcccgaggaggaggctccc
cggccccagctggacctgcaggcctccaaaaagctgccagatctctatggcaatccaccccaagagctcatcggagagc
ccctggaggacctggaccccttctatagcacccaaaagactttcatcgtactgaataaaggcaagaccatcttccggttcagt
gccaccaacgccttgtatgtcctcagtcccttccaccccatccggagagcggctgtgaagattctggttcactcgctcttcaa
catgctcatcatgtgcaccatcctcaccaactgcgtgttcatggcccagcacgaccctccaccctggaccaagtatgtcgag
tacaccttcaccgccatttacacctttgagtctctggtcaagattctggctcgaggcttctgcctgcacgcgttcactttccttcg
ggacccatggaactggctggactttagtgtgattatcatggcatacacaactgaatttgtggacctgggcaatgtctcagcctt
acgcaccttccgagtcctccgggccctgaaaactatatcagtcatttcagggctgaagaccatcgtgggggccctgatcca
gtctgtgaagaagctggctgatgtgatggtcctcacagtcttctgcctcagcgtctttgccctcatcggcctgcagctcttcat
gggcaacctaaggcacaagtgcgtgcgcaacttcacagcgctcaacggcaccaacggctccgtggaggccgacggctt
ggtctgggaatccctggacctttacctcagtgatccagaaaattacctgctcaagaacggcacctctgatgtgttactgtgtg
ggaacagctctgacgctgggacatgtccggagggctaccggtgcctaaaggcaggcgagaaccccgaccacggctaca
ccagcttcgattcctttgcctgggcctttcttgcactcttccgcctgatgacgcaggactgctgggagcgcctctatcagcag
accctcaggtccgcagggaagatctacatgatcttcttcatgcttgtcatcttcctggggtccttctacctggtgaacctgatcc
tggccgtggtcgcaatggcctatgaggagcaaaaccaagccaccatcgctgagaccgaggagaaggaaaagcgcttcc
aggaggccatggaaatgctcaagaaagaacacgaggccctcaccatcaggggtgtggataccgtgtcccgtagctccttg
gagatgtcccctttggccccagtaaacagccatgagagaagaagcaagaggagaaaacggatgtcttcaggaactgagg
 
agtgtggggaggacaggctccccaagtctgactcagaagatggtcccagagcaatgaatcatctcagcctcacccgtggc
ctcagcaggacttctatgaagccacgttccagccgcgggagcattttcacctttcgcaggcgagacctgggttctgaagca
gattttgcagatgatgaaaacagcacagcgggggagagcgagagccaccacacatcactgctggtgccctggcccctgc
gccggaccagtgcccagggacagcccagtcccggaacctcggctcctggccacgccctccatggcaaaaagaacagc
actgtggactgcaatggggtggtctcattactgggggcaggcgacccagaggccacatccccaggaagccacctcctcc
gccctgtgatgctagagcacccgccagacacgaccacgccatcggaggagccaggcgggccccagatgctgacctccc
aggctccgtgtgtagatggcttcgaggagccaggagcacggcagcgggccctcagcgcagtcagcgtcctcaccagcg
cactggaagagttagaggagtctcgccacaagtgtccaccatgctggaaccgtctcgcccagcgctacctgatctgggagt
gctgcccgctgtggatgtccatcaagcagggagtgaagttggtggtcatggacccgtttactgacctcaccatcactatgtg
catcgtactcaacacactcttcatggcgctggagcactacaacatgacaagtgaattcgaggagatgctgcaggtcggaaa
cctggtcttcacagggattttcacagcagagatgaccttcaagatcattgccctcgacccctactactacttccaacagggct
ggaacatcttcgacagcatcatcgtcatccttagcctcatggagctgggcctgtcccgcatgagcaacttgtcggtgctgcg
ctccttccgcctgctgcgggtcttcaagctggccaaatcatggcccaccctgaacacactcatcaagatcatcgggaactca
gtgggggcactggggaacctgacactggtgctagccatcatcgtgttcatctttgctgtggtgggcatgcagctctttggcaa
gaactactcggagctgaggacagcgactcaggcctgctgcctcgctggcacatgatggacttctttcatgccttcctcatc
atcttccgcatcctctgtggagagtggatcgagaccatgtgggactgcatggaggtgtcggggcagtcattatgcctgctgg
tcttcttgcttgttatggtcattggcaaccttgtggtcctgaatctcttcctggccttgctgctcagctccttcagtgcagacaacc
tcacagcccctgatgaggacagagagatgaacaacctccagctggccctggcccgcatccagaggggcctgcgctttgt
caagcggaccacctgggatttctgctgtggtctcctgcggcagcggcctcagaagcccgcagcccttgccgcccagggc
cagctgcccagctgcattgccacccccctactccccgccacccccagagacggagaaggtgcctcccacccgcaaggaa
acacggtttgaggaaggcgagcaaccaggccagggcacccccggggatccagagcccgtgtgtgtgcccatcgctgtg
gccgagtcagacacagatgaccaagaagaagatgaggagaacagcctgggcacggaggaggagtccagcaagcagc
aggaatcccagcctgtgtccggtggcccagaggcccctccggattccaggacctggagccaggtgtcagcgactgcctc
ctctgaggccgaggccagtgcatctcaggccgactggcggcagcagtggaaagcggaaccccaggccccagggtgcg
gtgagaccccagaggacagttgctccgagggcagcacagcagacatgaccaacaccgctgagctcctggagcagatcc
ctgacctcggccaggatgtcaaggacccagaggactgcttcactgaaggctgtgtccggcgctgtccctgctgtgcggtg
gacaccacacaggccccagggaaggtctggtggcggttgcgcaagacctgctaccacatcgtggagcacagctggttcg
agacattcatcatcttcatgatcctactcagcagtggagcgctggccttcgaggacatctacctagaggagcggaagaccat
caaggttctgcttgagtatgccgacaagatgttcacatatgtcttcgtgctggagatgctgctcaagtgggtggcctacggctt
caagaagtacttcaccaatgcctggtgctggctcgacttcctcatcgtagacgtctctctggtcagcctggtggccaacaccc
tgggctttgccgagatggccccatcaagtcactgcggacgctgcgtgcactccgtcctctgagagctctgtcacgatttga
gggcatgagggtggtggtcaatgccctggtgggcgccatcccgtccatcatgaacgtcctcctcgtctgcctcatcttctgg
ctcatcttcagcatcatgggcgtgaacctctttgcggggaagtttgggaggtgcatcaaccagacagagggagacttgcctt
tgaactacaccatcgtgaacaacaagagccagtgtgagtccttgaacttgaccggagaattgtactggaccaaggtgaaag
tcaactttgacaacgtgggggccgggtacctggcccttctgcaggtggcaacatttaaaggctggatggacattatgtatgc
agctgtggactccagggggtatgaagagcagcctcagtgggaatacaacctctacatgtacatctattttgtcattttcatcat
ctttgggtctttcttcaccctgaacctctttattggtgtcatcattgacaacttcaaccaacagaagaaaaagttaggggggccag
gacatcttcatgacagaggagcagaagaagtactacaatgccatgaagaagctgggctccaagaagccccagaagccca
tcccacggcccctgaacaagtaccagggcttcatattcgacattgtgaccaagcaggcctttgacgtcaccatcatgtttctg
atctgcttgaatatggtgaccatgatggtggagacagatgaccaaagtcctgagaaaatcaacatcttggccaagatcaacc
tgctctttgtggccatcttcacaggcgagtgtattgtcaagctggctgccctgcgccactactacttcaccaacagctggaata
tcttcgacttcgtggttgtcatcctctccatcgtgggcactgtgctctcggacatcatccagaagtacttcttctccccgacgct
cttccgagtcatccgcctggcccgaataggccgcatcctcagactgatccgaggggccaaggggatccgcacgctgctct
ttgccctcatgatgtccctgcctgccctcttcaacatcgggctgctgctcttcctcgtcatgttcatctactccatctttggcatgg
ccaacttcgcttatgtcaagtgggaggctggcatcgacgacatgttcaacttccagaccttcgccaacagcatgctgtgcctc
ttccagatcaccacgtcggccggctgggatggcctcctcagccccatcctcaacactgggccgccctactgcgacccccac
tctgcccaacagcaatggctctcggggggactgcgggagcccagccgtgggcatcctcttcttcaccacctacatcatcat
ctccttcctcatcgtggtcaacatgtacattgccatcatcctggagaacttcagcgtggccacggaggagagcaccgagcc
cctgagtgaggacgacttcgatatgttctatgagatctgggagaaatttgacccagaggccactcagtttattgagtattcggt
cctgtctgactttgccgatgccctgtctgagccactccgtatcgccaagcccaaccagataagcctcatcaacatggacctg
 
cccatggtgagtggggaccgcatccattgcatggacattctctttgccttcaccaaaagggtcctgggggagtctggggag
atggacgccctgaagatccagatggaggagaagttcatggcagccaacccatccaagatctcctacgagcccatcaccac
cacactccggcgcaagcacgaagaggtgtcggccatggttatccagagagccttccgcaggcacctgctgcaacgctctt
tgaagcatgcctccttcctcttccgtcagcaggcgggcagcggcctctccgaagaggatgcccctgagcgagagggcctc
atcgcctacgtgatgagtgagaacttctcccgaccccttggcccaccctccagctcctccatctcctccacttccttcccaccc
tcctatgacagtgtcactagagccaccagcgataacctccaggtgcgggggtctgactacagccacagtgaagatctcgc
cgacttcccccccttctccggacagggaccgtgagtccatcgtgtga (SEQ ID NO: 10)
H.s. SCN7A 
atgttggcttcaccagaacctaagggccttgttcccttcactaaagagtcttttgaacttataaaacagcatattgctaaaacac
ataatgaagaccatgaagaagaagacttaaagccaactcctgatttggaagttggcaaaaagcttccatttatttatggaaac
ctttctcaaggaatggtgtcagagcccttggaagatgtggacccatattactacaagaaaaaaatactttcatagtattaaata
aaaatagaacaatcttcagattcaatgcggcttccatcttgtgtacattgtctcctttcaattgtattagaagaacaactatcaag
gttttggtacatcccttttccaactgtttattctaattagtgtcctgattgattgcgtattcatgtccctgactaatttgccaaaatgg
agaccagtattagagaatactttgcttggaatttacacatttgaaatacttgtaaaactctttgcaagaggtgtctgggcaggat
cattttccttcctcggtgatccatggaactggctcgatttcagcgtaactgtgtttgaggttattataagatactcacctctggact
tcattccaacgcttcaaactgcaagaactttgagaattttaaaaattattcctttaaatcaaggtctgaaatcccttgtaggggtc
ctgatccactgcttgaagcagcttattggtgtcattatcctaactctgtttttctgagcatatttctctaattgggatggggctctt
catgggcaacttgaaacataaatgttttcgatggccccaagagaatgaaaatgaaaccctgcacaacagaactggaaaccc
atattatattcgagaaacagaaaacttttattatttggaaggagaaagatatgctctcctttgtggcaacaggacagatgctggt
cagtgtcctgaaggatatgtgtgtgtaaaagctggcataaatcctgatcaaggcttcacaaattttgacagttttggctgggcc
ttatttgccctatttcggttaatggctcaggattaccctgaagtactttatcaccagatactttatgcttctgggaaggtctacatg
atatttttgtggtggtaagtttttgttttcctttatatggcaagtttgttcttaggcatacttgccatggcctatgaagaagaaaag
cagagagttggtgaaatatctaagaagattgaaccaaaatttcaacagactggaaaagaacttcaagaaggaaatgaaaca
gatgaggccaagaccatacaaatagaaatgaagaaaaggtcaccaatttccacagacacatcattggatgtgttggaagat
gctactctcagacataaggaagaacttgaaaaatccaagaagatatgcccattatactggtataagtttgctaaaactttcttga
tctggaattgttctccctgttggttaaaattgaaagagtttgtccataggattataatggcaccatttactgatcttttccttatcata
tgcataattttaaacgtatgttttctgaccttggagcattatccaatgagtaaacaaactaacactcttctcaacattggaaacct
ggttttcattggaattttcacagcagaaatgattttaaaaataattgcaatgcatccatatgggtatttccaagtaggttggaacat
ttttgatagcatgatagtgttccatggtttaatagaactttgtctagcaaatgttgcaggaatggctcttcttcgattattcaggatg
ttaagaattttcaagttgggaaagtattggccaacattccagattttgatgtggtctcttagtaactcatgggtggccctgaaag
acttggtcctgttgttgttcacattcatcttcttttctgctgcattcggcatgaagctgtttggtaagaattatgaagaatttgtctgc
cacatagacaaagactgtcaactcccacgctggcacatgcatgactttttccactccttcctgaatgtgttccgaattctctgtg
gagagtgggtagagaccttgtgggactgtatggaggttgcaggccaatcctggtgtattcctttttacctgatggtcattttaat
tggaaatttactggtactttacctgtttctggcattggtgagctcatttagttcatgcaaggatgtaacagctgaagagaataatg
aagcaaaaaatctccagcttgcagtggcaagaattaaaaaggaataaactatgtgcttcttaaaatactatgcaaaacacaa
aatgtcccaaaggacacaatggaccatgtaaatgaggtatatgttaaagaagatatttctgaccataccctttctgaattgagc
aacacccaagatttctcaaagataaggaaaaaagcagtggcacagagaaaaacgctactgaaaatgagagccaatcact
tatccccagtcctagtgtctcagaaactgtaccaattgcttcaggagaatctgatatagaaaatctggataataaggagattca
gagtaagtctggtgatggaggcagcaaagagaaaataaagcaatctagctcatctgaatgcagtactgttgatattgctatct
ctgaagaagaagaaatgttctatggaggtgaaagatcaaagcatctgaaaaatggttgcagacgcggatcttcacttggtca
aatcagtggagcatccaagaaaggaaaaatctggcagaacatcaggaaaacctgctgcaagattgtagagaacaattggtt
taagtgttttattgggcttgttactctgctcagcactggcactctggcttttgaagatatatatatggatcagagaaagacaatta
aaattttattagaatatgctgacatgatctttacttatatcttcattctggaaatgcttctaaaatggatggcatatggttttaaggcc
tatttctctaatggctggtacaggctggacttcgtggttgttattgtgttttgtcttagcttaataggcaaaactcgggaagaacta
aaacctcttatttccatgaaattccttcggcccctcagagttctatctcaatttgaaagaatgaaggtggttgtgagagctttgat
caaaacaaccttacccactttgaatgtgtttcttgtctgcctgatgatctggctgattttagtatcatgggagtagacttatttgct
ggcagattctatgaatgcattgacccaacaagtggagaaaggtttccttcatctgaagtcatgaataagagtcggtgtgaaag
 
ccttctgtttaacgaatccatgctatgggaaaatgcaaaaatgaactttgataatgttggaaatggtttcctttctctgcttcaagt
agcaacatttaatggatggatcactattatgaattcagcaattgattctgttgctgttaatatacagcctcattttgaagtcaacat
ctacatgtattgttactttatcaactttattatatttggagtatttctccctctgagtatgctgattactgttattattgataatttcaaca
agcataaaataaagctgggaggctcaaatatctttataacggttaaacagagaaaacagtaccgcaggctgaagaagctaa
tgtatgaggattctcaaagaccagtacctcgcccattaaacaagctccaaggattcatctttgatgtggtaacaagccaagctt
ttaatgtcattgttatggttcttatatgtttccaagcaatagccatgatgatagacactgatgttcagagtctacaaatgtccattg
ctctctactggattaactcaattttgttatgctatatactatggaatgtatactgaagctcatcgctttccgttgtttttatttcaccat
tgcgtggaacatttttgattttatggtggttatttctccatcacaggactatgtctgcctatgacagtaggatcctaccttgtgcct
ccttcacttgtgcaactgatacttctctcacggatcattcacatgctgcgtcttggaaaaggaccaaaggtgtttcataatctgat
gcttcctttgatgctgtccctcccagcattattgaacatcattcttctcatcttcctggtcatgttcatctatgccgtatttggaatgt
ataattttgcctatgttaaaaaagaagctggaattaatgatgtgtctaattttgaaacctttggcaacagtatgctctgtctttttca
agttgcaatatttgctggttgggatgggatgcttgatgcaattttcaacagtaaatggtctgactgtgatcctgataaaattaacc
ctgggactcaagttagaggagattgtgggaacccctctgttgggatttttattttgtcagttatatcctcatatcatggctgatca
ttgtaaatatgtacattgttgttgtcatggagtttttaaatattgcttctaagaagaaaaacaagaccttgagtgaagatgatttag
gaaattctttcaggtatggaaaaggtttgatcctgataggacccagtacatagactctagcaagctttcagattttgcagctgct
cttgatcctcctcttttcatggcaaaaccaaacaagggccagctcattgctttggacctccccatggctgttggggacagaatt
cattgcctcgatatcttacttgcttttacaaagagagttatgggtcaagatgtgaggatggagaaagttgtttcagaaatagaat
cagggtttttgttagccaacccttttaagatcacatgtgagccaattacgactactttgaaacgaaaacaagaggcagtttcag
caaccatcattcaacgtgcttataaaaattaccgcttgaggcgaaatgacaaaaatacatcagatattcatatgatagatggtg
acagagatgttcatgctactaaagaaggtgcctatttgacaaagctaaggaaaagtcacctattcaaagccagatctaa
(SEQ ID NO: 11)
H.s. SCN8A 
atggcagcgcggctgcttgcaccaccaggccctgatagtttcaagcctttcacccctgagtcactggcaaacattgagagg
cgcattgctgagagcaagctcaagaaaccaccaaaggccgatggcagtcatcgggaggacgatgaggacagcaagcc
caagccaaacagcgacctggaagcagggaagagtttgcctttcatctacggggacatcccccaaggcctggttgcagttc
ccctggaggactttgacccatactatttgacgcagaaaacctttgtagtattaaacagagggaaaactctcttcagatttagtg
ccacgcctgccttgtacattttaagtccttttaacctgataagaagaatagctattaaaattttgatacattcagtatttagcatgat
cattatgtgcactattttgaccaactgtgtattcatgacttttagtaaccctcctgactggtcgaagaatgtggagtacacgttca
cagggatttatacatttgaatcactagtgaaaatcattgcaagaggtttctgcatagatggctttacctttttacgggacccatgg
aactggttagatttcagtgtcatcatgatggcgtatataacagagtttgtaaacctaggcaatgtttcagctctacgcactttcag
ggtactgagggctttgaaaactatttcggtaatcccaggcctgaagacaattgtgggtgccctgattcagtctgtgaagaaac
tgtcagatgtgatgatcctgacagtgttctgcctgagtgtttttgccttgatcggactgcagctgttcatggggaaccttcgaaa
caagtgtgttgtgtggcccataaacttcaacgagagctatcttgaaaatggcaccaaaggctttgattgggaagagtatatca
acaataaaacaaatttctacacagttcctggcatgctggaacctttactctgtgggaacagttctgatgctgggcaatgccca
gagggataccagtgtatgaaagcaggaaggaaccccaactatggttacacaagttttgacacttttagctgggccttcttggc
attatttcgccttatgacccaggactattgggaaaacttgtatcaattgactttacgagcagccgggaaaacatacatgatcttc
ttcgtcttggtcatctttgtgggttctttctatctggtgaacttgatcttggctgtggtggccatggcttatgaagaacagaatcag
gcaacactggaggaggcagaacaaaaagaggctgaatttaaagcaatgttggagcaacttaagaagcaacaggaagag
gcacaggctgctgcgatggccacttcagcaggaactgtctcagaagatgccatagaggaagaaggtgaagaaggaggg
ggctcccctcggagctcttctgaaatctctaaactcagctcaaagagtgcaaaggaaagacgtaacaggagaaagaagag
gaagcaaaaggaactctctgaaggagaggagaaaggggatcccgagaaggtgtttaagtcagagtcagaagatggcat
gagaaggaaggcctttcggctgccagacaacagaatagggaggaaattttccatcatgaatcagtcactgctcagcatccc
aggctcgcccttcctctcccgccacaacagcaagagcagcatcttcagtttcaggggacctgggcggttccgagacccgg
gctccgagaatgagttcgcggatgacgagcacagcacggtggaggagagcgagggccgccgggactccctcttcatcc
ccatccgggcccgcgagcgccggagcagctacagcggctacagcggctacagccagggcagccgctcctcgcgcatc
ttccccagcctgcggcgcagcgtgaagcgcaacagcacggtggactgcaacggcgtggtgtccctcatcggcggcccc
ggctcccacatcggcgggcgtctcctgccagaggctacaactgaggtggaaattaagaagaaaggccctggatctctttta
 
gtttccatggaccaattagcctcctacgggcggaaggacagaatcaacagtataatgagtgttgttacaaatacactagtaga
agaactggaagagtctcagagaaagtgcccgccatgctggtataaatttgccaacactttcctcatctgggagtgccacccc
tactggataaaactgaaagagattgtgaacttgatagttatggacccttttgtggatttagccatcaccatctgcatcgtcctga
atacactgtttatggcaatggagcaccatcctatgacaccacaatttgaacatgtcttggctgtaggaaatctggttttcactgg
aattttcacagcggaaatgttcctgaagctcatagccatggatccctactattatttccaagaaggttggaacatttttgacgga
tttattgtctccctcagtttaatggaactgagtctagcagacgtggaggggctttcagtgctgcgatctttccgattgctccgag
tcttcaaattggccaaatcctggcccaccctgaacatgctaatcaagattattggaaattcagtgggtgccctgggcaacctg
acactggtgctggccattattgtcttcatctttgccgtggtggggatgcaactctttggaaaaagctacaaagagtgtgtctgc
aagatcaaccaggactgtgaactccctcgctggcatatgcatgactttttccattccttcctcattgtctttcgagtgttgtgcgg
ggagtggattgagaccatgtgggactgcatggaagtggcaggccaggccatgtgcctcattgtctttatgatggtcatggtg
attggcaacttggtggtgctgaacctgtttctggccttgctcctgagctccttcagtgcagacaacctggctgccacagatga
cgatgggaaatgaacaacctccagatctcagtgatccgtatcaagaagggtgtggcctggaccaaactaaaggtgcacg
ccttcatgcaggcccactttaagcagcgtgaggctgatgaggtgaagcctctggatgagttgtatgaaaagaaggccaact
gtatcgccaatcacaccggtgcagacatccaccggaatggtgacttccagaagaatggcaatggcacaaccagcggcatt
ggcagcagcgtggagaagtacatcattgatgaggaccacatgtccttcatcaacaaccccaacttgactgtacgggtaccc
attgctgtgggcgagtctgactttgagaacctcaacacagaggatgttagcagcgagtcggatcctgaaggcagcaaagat
aaactagatgacaccagctcctctgaaggaagcaccattgatatcaaaccagaagtagaagaggtccctgtggaacagcc
tgaggaatacttggatccagatgcctgcttcacagaaggttgtgtccagcggttcaagtgctgccaggtcaacatcgaggaa
gggctaggcaagtcttggtggatcctgcggaaaacctgcttcctcatcgtggagcacaactggtttgagaccttcatcatctt
catgattctgctgagcagtggcgccctggccttcgaggacatctacattgagcagagaaagaccatccgcaccatcctgga
atatgctgacaaagtcttcacctatatcttcatcctggagatgttgctcaagtggacagcctatggcttcgtcaagttcttcacca
atgcctggtgttggctggacttcctcattgtggctgtctctttagtcagccttatagctaatgccctgggctactcggaactagg
tgccataaagtcccttaggaccctaagagctttgagacccttaagagccttatcacgatttgaaggatgagggtggtggtg
aatgccttggtgggcgccatcccctccatcatgaatgtgctgctggtgtgtctcatcttctggctgattttcagcatcatgggag
ttaacttgtttgcgggaaagtaccactactgctttaatgagacttctgaaatccgatttgaaattgaagatgtcaacaataaaact
gaatgtgaaaagcttatggaggggaacaatacagagatcagatggaagaacgtgaagatcaactttgacaatgttggggc
aggatacctggcccttcttcaagtagcaaccttcaaaggctggatggacatcatgtatgcagctgtagattcccggaagcct
gatgagcagcctaagtatgaggacaatatctacatgtacatctattttgtcatcttcatcatcttcggctccttcttcaccctgaac
ctgttcattggtgtcatcattgataacttcaatcaacaaaagaaaaagttcggaggtcaggacatcttcatgaccgaagaaca
gaagaagtactacaatgccatgaaaaagctgggctcaaagaagccacagaaacctattccccgccccttgaacaaaatcc
aaggaatcgtctttgattttgtcactcagcaagcctttgacattgttatcatgatgctcatctgccttaacatggtgacaatgatg
gtggagacagacactcaaagcaagcagatggagaacatcctctactggattaacctggtgtttgttatcttcttcacctgtgag
tgtgtgctcaaaatgtttgcgttgaggcactactacttcaccattggctggaacatcttcgacttcgtggtagtcatcctctccat
tgtgggaatgttcctggcagatataattgagaaatactttgtttccccaaccctattccgagtcatccgattggcccgtattggg
cgcatcttgcgtctgatcaaaggcgccaaagggattcgtaccctgctctttgccttaatgatgtccttgcctgccctgttcaaca
tcggccttctgctcttcctggtcatgttcatcttctccattttgggatgtccaattttgcatatgtgaagcacgaggctggtatcg
atgacatgttcaactttgagacatttggcaacagcatgatctgcctgtttcaaatcacaacctcagctggttgggatggcctgc
tgctgcccatcctaaaccgcccccctgactgcagcctagataaggaacacccagggagtggctttaagggagattgtggg
aacccctcagtgggcatcttcttctttgtaagctacatcatcatctctttcctaattgtcgtgaacatgtacattgccatcatcctg
gagaacttcagtgtagccacagaggaaagtgcagaccctctgagtgaggatgactttgagaccttctatgagatctgggag
aagttcgaccccgatgccacccagttcattgagtactgtaagctggcagactttgcagatgccttggagcatcctctccgagt
gcccaagcccaataccattgagctcatcgctatggatctgccaatggtgagcggggatcgcatccactgcttggacatcctt
tttgccttcaccaagcgggtcctgggagatagcggggagttggacatcctgcggcagcagatggaagagcggttcgtggc
atccaatccttccaaagtgtcttacgagccaatcacaaccacactgcgtcgcaagcaggaggaggtatctgcagtggtcct
gcagcgtgcctaccggggacatttggcaaggcggggcttcatctgcaaaaagacaacttctaataagctggagaatggag
gcacacaccgggagaaaaagagagcaccccatctacagcctccctcccgtcctatgacagtgtaactaaacctgaaaag
gagaaacagcagcgggcagaggaaggaagaagggaaagagccaaaagacaaaaagaggtcagagaatccaagtgtt
ag (SEQ ID NO: 12)
H.s. SCN9A 
atggcaatgttgcctcccccaggacctcagagctttgtccatttcacaaaacagtctcttgccctcattgaacaacgcattgct
gaaagaaaatcaaaggaacccaaagaagaaaagaaagatgatgatgaagaagccccaaagccaagcagtgacttggaa
gctggcaaacaactgcccttcatctatggggacattcctcccggcatggtgtcagagcccctggaggacttggacccctact
atgcagacaaaaagactttcatagtattgaacaaagggaaaacaatcttccgtttcaatgccacacctgctttatatatgctttct
cctttcagtcctctaagaagaatatctattaagattttagtacactccttattcagcatgctcatcatgtgcactattctgacaaact
gcatatttatgaccatgaataacccgccggactggaccaaaaatgtcgagtacacttttactggaatatatacttttgaatcact
tgtaaaaatccttgcaagaggcttctgtgtaggagaattcacttttcttcgtgacccgtggaactggctggattttgtcgtcattg
tttttgcgtatttaacagaatttgtaaacctaggcaatgtttcagctcttcgaactttcagagtattgagagctttgaaaactatttct
gtaatcccaggcctgaagacaattgtaggggctttgatccagtcagtgaagaagctttctgatgtcatgatcctgactgtgttc
tgtctgagtgtgtttgcactaattggactacagctgttcatgggaaacctgaagcataaatgttttcgaaattcacttgaaaataa
tgaaacattagaaagcataatgaataccctagagagtgaagaagactttagaaaatattttattacttggaaggatccaaaga
tgctctcctttgtggtttcagcacagattcaggtcagtgtccagaggggtacacctgtgtgaaaattggcagaaaccctgatta
tggctacacgagctttgacactttcagctgggccttcttagccttgtttaggctaatgacccaagattactgggaaaacctttac
caacagacgctgcgtgctgctggcaaaacctacatgatcttctttgtcgtagtgattttcctgggctccttttatctaataaacttg
atcctggctgtggttgccatggcatatgaagaacagaaccaggcaaacattgaagaagctaaacagaaagaattagaattt
caacagatgttagaccgtcttaaaaaagagcaagaagaagctgaggcaattgcagcggcagcggctgaatatacaagtat
taggagaagcagaattatgggcctctcagagagttcttctgaaacatccaaactgagctctaaaagtgctaaagaaagaag
aaacagaagaaagaaaaagaatcaaaagaagctctccagtggagaggaaaagggagatgctgagaaattgtcgaaatca
gaatcagaggacagcatcagaagaaaaagtttccaccttggtgtcgaagggcataggcgagcacatgaaaagaggttgtc
tacccccaatcagtcaccactcagcattcgtggctccttgttttctgcaaggcgaagcagcagaacaagtctttttagtttcaaa
ggcagaggaagagatataggatctgagactgaatttgccgatgatgagcacagcattttggagacaatgagagcagaag
gggctcactgtttgtgccccacagaccccaggagcgacgcagcagtaacatcagccaagccagtaggtccccaccaatg
ctgccggtgaacgggaaaatgcacagtgctgtggactgcaacggtgtggtctccctggttgatggacgctcagccctcatg
ctccccaatggacagcttctgccagagggcacgaccaatcaaatacacaagaaaaggcgttgtagttcctatctcctttcag
aggatatgctgaatgatcccaacctcagacagagagcaatgagtagagcaagcatattaacaaacactgtggaagaacttg
aagagtccagacaaaaatgtccaccttggtggtacagatttgcacacaaattcttgatctggaattgctctccatattggataa
aattcaaaaagtgtatctattttattgtaatggatccttttgtagatcttgcaattaccatttgcatagttttaaacacattatttatggc
tatggaacaccacccaatgactgaggaattcaaaaatgtacttgctataggaaatttggtctttactggaatctttgcagctgaa
atggtattaaaactgattgccatggatccatatgagtatttccaagtaggctggaatatttttgacagccttattgtgactttaagtt
tagtggagctctttctagcagatgtggaaggattgtcagttctgcgatcattcagactgctccgagtcttcaagttggcaaaat
cctggccaacattgaacatgctgattaagatcattggtaactcagtaggggctctaggtaacctcaccttagtgttggccatca
tcgtcttcatttttgctgtggtcggcatgcagctctttggtaagagctacaaagaatgtgtctgcaagatcaatgatgactgtac
gctcccacggtggcacatgaacgacttcttccactccttcctgattgtgttccgcgtgctgtgtggagagtggatagagacca
tgtgggactgtatggaggtcgctggtcaagctatgtgccttattgtttacatgatggtcatggtcattggaaacctggtggtcct
aaacctatttctggccttattattgagctcatttagttcagacaatcttacagcaattgaagaagaccctgatgcaaacaacctc
cagattgcagtgactagaattaaaaagggaataaattatgtgaaacaaaccttacgtgaatttattctaaaagcattttccaaaa
agccaaagatttccagggagataagacaagcagaagatctgaatactaagaaggaaaactatatttctaaccatacacttgc
tgaaatgagcaaaggtcacaatttcctcaaggaaaaagataaaatcagtggttttggaagcagcgtggacaaacacttgatg
gaagacagtgatggtcaatcatttattcacaatcccagcctcacagtgacagtgccaattgcacctggggaatccgatttgga
aaatatgaatgctgaggaacttagcagtgattcggatagtgaatacagcaaagtgagattaaaccggtcaagctcctcagag
tgcagcacagttgataaccctttgcctggagaaggagaagaagcagaggctgaacctatgaattccgatgagccagaggc
ctgtttcacagatggttgtgtacggaggttctcatgctgccaagttaacatagagtcagggaaaggaaaaatctggtggaac
atcaggaaaacctgctacaagattgttgaacacagttggtttgaaagcttcattgtcctcatgatcctgctcagcagtggtgcc
ctggcttttgaagatatttatattgaaaggaaaaagaccattaagattatcctggagtatgcagacaagatcttcacttacatctt
cattctggaaatgcttctaaaatggatagcatatggttataaaacatatttcaccaatgcctggtgttggctggatttcctaattgt
tgatgtttctttggttactttagtggcaaacactcttggctactcagatcttggccccattaaatcccttcggacactgagagcttt
aagacctctaagagccttatctagatttgaaggaatgagggtcgttgtgaatgcactcataggagcaattccttccatcatgaa
tgtgctacttgtgtgtcttatattctggctgatattcagcatcatgggagtaaatttgtttgctggcaagttctatgagtgtattaac
 
accacagatgggtcacggtttcctgcaagtcaagttccaaatcgttccgaatgttttgcccttatgaatgttagtcaaaatgtgc
gatggaaaaacctgaaagtgaactttgataatgtcggacttggttacctatctctgcttcaagttgcaacttttaagggatggac
gattattatgtatgcagcagtggattctgttaatgtagacaagcagcccaaatatgaatatagcctctacatgtatatttattttgt
cgtctttatcatctttgggtcattcttcactttgaacttgttcattggtgtcatcatagataatttcaaccaacagaaaaagaagctt
ggaggtcaagacatctttatgacagaagaacagaagaaatactataatgcaatgaaaaagctggggtccaagaagccaca
aaagccaattcctcgaccagggaacaaaatccaaggatgtatatttgacctagtgacaaatcaagcctttgatattagtatcat
ggttcttatctgtctcaacatggtaaccatgatggtagaaaaggagggtcaaagtcaacatatgactgaagttttatattggata
aatgtggtttttataatccttttcactggagaatgtgtgctaaaactgatctccctcagacactactacttcactgtaggatggaat
atttttgattttgtggttgtgattatctccattgtaggtatgttctagctgatttgattgaaacgtattttgtgtcccctaccctgttcc
gagtgatccgtcttgccaggattggccgaatcctacgtctagtcaaaggagcaaaggggatccgcacgctgctctttgcttt
gatgatgtcccttcctgcgttgtttaacatcggcctcctgctcttcctggtcatgttcatctacgccatctttggaatgtccaacttt
gcctatgttaaaaaggaagatggaattaatgacatgttcaattttgagacctttggcaacagtatgatttgcctgttccaaattac
aacctctgctggctgggatggattgctagcacctattcttaacagtaagccacccgactgtgacccaaaaaaagttcatcctg
gaagttcagttgaaggagactgtggtaacccatctgttggaatattctactttgttagttatatcatcatatccttcctggttgtggt
gaacatgtacattgcagtcatactggagaattttagtgttgccactgaagaaagtactgaacctctgagtgaggatgactttga
gatgttctatgaggtttgggagaagtttgatcccgatgcgacccagtttatagagttctctaaactctctgattttgcagctgccc
tggatcctcctcttctcatagcaaaacccaacaaagtccagctcattgccatggatctgcccatggttagtggtgaccggatc
cattgtcttgacatcttatttgcttttacaaagcgtgttttgggtgagagtggggagatggattctcttcgttcacagatggaaga
aaggttcatgtctgcaaatccttccaaagtgtcctatgaacccatcacaaccacactaaaacggaaacaagaggatgtgtct
gctactgtcattcagcgtgcttatagacgttaccgcttaaggcaaaatgtcaaaaatatatcaagtatatacataaaagatgga
gacagagatgatgatttactcaataaaaaagatatggcttttgataatgttaatgagaactcaagtccagaaaaaacagatgc
cacttcatccaccacctctccaccttcatatgatagtgtaacaaagccagacaaagagaaatatgaacaagacagaacaga
aaaggaagacaaagggaaagacagcaaggaaagcaaaaaatag (SEQ ID NO: 13)
H.s. SCN10A 
atggaattccccattggatccctcgaaactaacaacttccgtcgctttactccggagtcactggtggagatagagaagcaaat
tgctgccaagcagggaacaaagaaagccagagagaagcatagggagcagaaggaccaagaagagaagcctcggccc
cagctggacttgaaagcctgcaaccagctgcccaagttctatggtgagctcccagcagaactgatcggggagcccctgga
ggatctagatccgttctacagcacacaccggacatttatggtgctgaacaaagggaggaccatttcccggtttagtgccact
cgggccctgtggctattcagtcctttcaacctgatcagaagaacggccatcaaagtgtctgtccactcgtggttcagtttattta
ttacggtcactattttggttaattgtgtgtgcatgacccgaactgaccttccagagaaaattgaatatgtcttcactgtcatttaca
cctttgaagccttgataaagatactggcaagaggattttgtctaaatgagttcacgtacctgagagatccttggaactggctgg
attttagcgtcattaccctggcatatgttggcacagcaatagatctccgtgggatctcaggcctgcggacattcagagttctta
gagcattaaaaacagtttctgtgatcccaggcctgaaggtcattgtgggggccctgattcactcagtgaagaaactggctga
tgtgaccatcctcaccatcttctgcctaagtgtttttgccttggtggggctgcaactcttcaagggcaacctcaaaaataaatgt
gtcaagaatgacatggctgtcaatgagacaaccaactactcatctcacagaaaaccagatatctacataaataagcgaggc
acttctgaccccttactgtgtggcaatggatctgactcaggccactgccctgatggttatatctgccttaaaacttctgacaacc
cggattttaactacaccagctttgattcctttgcttgggctttcctctcactgttccgcctcatgacacaggattcctgggaacgc
ctctaccagcagaccctgaggacttctgggaaaatctatatgatctttttgtgctcgtaatcttcctgggatctttctacctggtc
aacttgatcttggctgtagtcaccatggcgtatgaggagcagaaccaggcaaccactgatgaaattgaagcaaaggagaa
gaagttccaggaggccctcgagatgctccggaaggagcaggaggtgctagcagcactagggattgacacaacctctctc
cactcccacaatggatcacctttaacctccaaaaatgccagtgagagaaggcatagaataaagccaagagtgtcagaggg
ctccacagaagacaacaaatcaccccgctctgatccttacaaccagcgcaggatgtcttttctaggcctcgcctctggaaaa
cgccgggctagtcatggcagtgtgttccatttccggtcccctggccgagatatctcactccctgagggagtcacagatgatg
gagtctttcctggagaccacgaaagccatcggggctctctgctgctgggtgggggtgctggccagcaaggccccctccct
agaagccctcttcctcaacccagcaaccctgactccaggcatggagaagatgaacaccaaccgccgcccactagtgagct
tgcccctggagctgtcgatgtctcggcattcgatgcaggacaaaagaagactttcttgtcagcagaatacttagatgaacctttt
ccgggcccaaagggcaatgagtgttgtcagtatcataacctccgtccttgaggaactcgaggagtctgaacagaagtgccc
 
accctgcttgaccagcttgtctcagaagtatctgatctgggattgctgccccatgtgggtgaagctcaagacaattctctttgg
gcttgtgacggatccctttgcagagctcaccatcaccttgtgcatcgtggtgaacaccatcttcatggccatggagcaccatg
gcatgagccctaccttcgaagccatgctccagataggcaacatcgtctttaccatatttttactgctgaaatggtcttcaaaat
cattgccttcgacccatactattatttccagaagaagtggaatatctttgactgcatcatcgtcactgtgagtctgctagagctg
ggcgtggccaagaaggaagcctgtctgtgctgcggagcttccgcttgctgcgcgtattcaagctggccaaatcctggcc
caccttaaacacactcatcaagatcatcggaaactcagtgggggcactggggaacctcaccatcatcctggccatcattgtc
tttgtctttgctctggttggcaagcagctcctaggggaaaactaccgtaacaaccgaaaaaatatctccgcgccccatgaag
actggccccgctggcacatgcacgacttcttccactctttcctcattgtcttccgtatcctctgtggagagtggattgagaacat
gtgggcctgcatggaagttggccaaaaatccatatgcctcatcctttcttgacggtgatggtgctagggaacctggtggtgc
ttaacctgttcatcgccctgctattgaactctttcagtgctgacaacctcacagccccggaggacgatggggaggtgaacaa
cctgcaggtggccctggcacggatccaggtctttggccatcgtaccaaacaggctctttgcagcttcttcagcaggtcctgc
ccattcccccagcccaaggcagagcctgagctggtggtgaaactcccactctccagctccaaggctgagaaccacattgc
tgccaacactgccaggggggagctctggagggctccaagctcccagaggccccagggatgagcacagtgacttcatcgct
aatccgactgtgtgggtctctgtgcccattgctgagggtgaatctgatcttgatgacttggaggatgatggtggggaagatgc
tcagagcttccagcaggaagtgatccccaaaggacagcaggagcagctgcagcaagtcgagaggtgtggggaccacct
gacacccaggagcccaggcactggaacatcttctgaggacctggctccatccctgggtgagacgtggaaagatgagtctg
ttcctcaggtccctgctgagggagtggacgacacaagctcctctgagggcagcacggtggactgcctagatcctgaggaa
atcctgaggaagatccctgagctggcagatgacctggaagaaccagatgactgcttcacagaaggatgcattcgccactgt
ccctgctgcaaactggataccaccaagagtccatgggatgtgggctggcaggtgcgcaagacttgctaccgtatcgtgga
gcacagctggtttgagagcttcatcatcttcatgatcctgctcagcagtggatctctggcctttgaagactattacctggacca
gaagcccacggtgaaagctttgctggagtacactgacagggtcttcacctttatctttgtgttcgagatgctgcttaagtgggt
ggcctatggcttcaaaaagtacttcaccaatgcctggtgctggctggacttcctcattgtgaatatctcactgataagtctcaca
gcgaagattctggaatattctgaagtggctcccatcaaagcccttcgaacccttcgcgctctgcggccactgcgggctctttc
tcgatttgaaggcatgcgggtggtggtggatgccctggtgggcgccatcccatccatcatgaatgtcctcctcgtctgcctca
tcttctggctcatcttcagcatcatgggtgtgaacctcttcgcagggaagttttggaggtgcatcaactataccgatggagagt
tttcccttgtacctttgtcgattgtgaataacaagtctgactgcaagattcaaaactccactggcagcttcttctgggtcaatgtg
aaagtcaactttgataatgttgcaatgggttaccttgcacttctgcaggtggcaacctttaaaggctggatggacattatgtatg
cagctgttgattcccgggaggtcaacatgcaacccaagtgggaggacaacgtgtacatgtatttgtactttgtcatcttcatca
tttttggaggcttcttcacactgaatctctttgttggggtcataattgacaacttcaatcaacagaaaaaaaagttagggggcca
ggacatcttcatgacagaggagcagaagaaatactacaatgccatgaagaagttgggctccaagaagccccagaagccc
atcccacggcccctgaacaagttccagggttttgtctttgacatcgtgaccagacaagcttttgacatcaccatcatggtcctc
atctgcctcaacatgatcaccatgatggtggagactgatgaccaaagtgaagaaaagacgaaaattctgggcaaaatcaac
cagttctttgtggccgtcttcacaggcgaatgtgtcatgaagatgttcgctttgaggcagtactacttcacaaatggctggaat
gtgtttgacttcattgtggtggttctctccattgcgagcctgatttttctgcaattcttaagtcacttcaaagttacttctccccaac
gctcttcagagtcatccgcctggcccgaattggccgcatcctcagactgatccgagcggccaaggggatccgcacactgc
tctttgccctcatgatgtccctgcctgccctcttcaacatcgggctgttgctattccttgtcatgttcatctactctatcttcggtatg
tccagctttccccatgtgaggtgggaggctggcatcgacgacatgttcaacttccagaccttcgccaacagcatgctgtgcc
tcttccagattaccacgtcggccggctgggatggcctcctcagccccatcctcaacacagggccccccctactgtgacccca
atctgcccaacagcaatggcaccagaggggactgtgggagcccagccgtaggcatcatcttcttcaccacctacatcatca
tctccttcctcatcatggtcaacatgtacattgcagtgattctggagaacttcaatgtggccacggaggagagcactgagccc
ctgagtgaggacgactttgacatgttctatgagacctgggagaagtttgacccagaggccactcagtttattaccttttctgctc
tctcggactttgcagacactctctctggtcccctgagaatcccaaaacccaatcgaaatatactgatccagatggacctgcctt
tggtccctggagataagatccactgcttggacatcctttttgctttcaccaagaatgtcctaggagaatccggggagttggatt
ctctgaaggcaaatatggaggagaagtttatggcaactaatctttcaaaatcatcctatgaaccaatagcaaccactctccgat
ggaagcaagaagacatttcagccactgtcattcaaaaggcctatcggagctatgtgctgcaccgctccatggcactctctaa
caccccatgtgtgcccagagctgaggaggaggctgcatcactcccagatgaaggttttgttgcattcacagcaaatgaaaat
tgtgtactcccagacaaatctgaaactgcttctgccacatcattcccaccgtcctatgagagtgtcactagaggccttagtgat
agagtcaacatgaggacatctagctcaatacaaaatgaagatgaagccaccagtatggagctgattgcccctgggccctag
(SEQ ID NO: 14)
H.s. SCN11A 
atggatgacagatgctacccagtaatctttccagatgagcggaatttccgccccttcacttccgactctctggctgcaattgag
aagcggattgccatccaaaaggagaaaaagaagtctaaagaccagacaggagaagtaccccagcctcggcctcagcttg
acctaaaggcctccaggaagttgcccaagctctatggcgacattcctcgtgagctcataggaaagcctctggaagacttgg
acccattctaccgaaatcataagacatttatggtgttaaacagaaagaggacaatctaccgcttcagtgccaagcatgccttg
ttcatttttgggcctttcaattcaatcagaagtttagccattagagtctcagtccattcattgttcagcatgttcattatcggcaccg
ttatcatcaactgcgtgttcatggctacagggcctgctaaaaacagcaacagtaacaatactgacattgcagagtgtgtcttca
ctgggatttatatttttgaagctttgattaaaatattggcaagaggtttcattctggatgagttttctttccttcgagatccatggaac
tggctggactccattgtcattggaatagcgattgtgtcatatattccaggaatcaccatcaaactattgcccctgcgtaccttcc
gtgtgttcagagctttgaaagcaatttcagtagtttcacgtctgaaggtcatcgtgggggccttgctacgctctgtgaagaagc
tggtcaacgtgattatcctcaccttcttttgcctcagcatctttgccctggtaggtcagcagctcttcatgggaagtctgaacctg
aaatgcatctcgagggactgtaaaaatatcagtaacccggaagcttatgaccattgctttgaaaagaaagaaaattcacctga
attcaaaatgtgtggcatctggatgggtaacagtgcctgttccatacaatatgaatgtaagcacaccaaaattaatcctgacta
taattatacgaattttgacaactttggctggtcttttcttgccatgttccggctgatgacccaagattcctgggagaagctttatca
acagaccctgcgtactactgggctctactcagtcttcttcttcattgtggtcattttcctgggctccttctacctgattaacttaacc
ctggctgttgttaccatggcatatgaggagcagaacaagaatgtagctgcagagatagaggccaaggaaaagatgtttcag
gaagcccagcagctgttaaaggaggaaaaggaggctctggttgccatgggaattgacagaagttcacttacttcccttgaa
acatcatattttaccccaaaaaagagaaagctctttggtaataagaaaaggaagtccttctttttgagagagtctgggaaagac
cagcctcctgggtcagattctgatgaagattgccaaaaaaagccacagctcctagagcaaaccaaacgactgtcccagaat
ctatcactggaccactttgatgagcatggagatcctctccaaaggcagagagcactgagtgctgtcagcatcctcaccatca
ccatgaaggaacaagaaaaatcacaagagccttgtctcccttgtggagaaaacctggcatccaagtacctcgtgtggaact
gttgcccccagtggctgtgcgttaagaaggtcctgagaactgtgatgactgacccgtttactgagctggccatcaccatctg
catcatcatcaacactgtcttcttggccatggagcatcacaagatggaggccagttttgagaagatgttgaatatagggaattt
ggtttcactagcatttttatagcagaaatgtgcctaaaaatcattgcgctcgatccctaccactactttcgccgaggctggaac
atttttgacagcattgttgctcttctgagttttgcagatgtaatgaactgtgtacttcaaaagagaagctggccattcttgcgttcc
ttcagagtgctcagggtcttcaagttagccaaatcctggccaactttgaacacactaattaagataatcggcaactctgtcgga
gcccttggaagcctgactgtggtcctggtcattgtgatctttattttctcagtagttggcatgcagctttttggccgtagcttcaat
tcccaaaagagtccaaaactctgtaacccgacaggcccgacagtctcatgtttacggcactggcacatgggggatttctgg
cactccttcctagtggtattccgcatcctctgcggggaatggatcgaaaatatgtgggaatgtatgcaagaagcgaatgcatc
atcatcattgtgtgttattgtcttcatattgatcacggtgataggaaaacttgtggtgctcaacctcttcattgccttactgctcaatt
cctttagcaatgaggaaagaaatggaaacttagaaggagaggccaggaaaactaaagtccagttagcactggatcgattc
cgccgggctttttgttttgtgagacacactcttgagcatttctgtcacaagtggtgcaggaagcaaaacttaccacagcaaaa
agaggtggcaggaggctgtgctgcacaaagcaaagacatcattcccctggtcatggagatgaaaaggggctcagagacc
caggaggagcttggtatactaacctctgtaccaaagaccctgggcgtcaggcatgattggacttggttggcaccacttgcg
gaggaggaagatgacgttgaattttctggtgaagataatgcacagcgcatcacacaacctgagcctgaacaacaggcctat
gagctccatcaggagaacaagaagcccacgagccagagagttcaaagtgtggaaattgacatgttctctgaagatgagcc
tcatctgaccatacaggatccccgaaagaagtctgatgttaccagtatactatcagaatgtagcaccattgatcttcaggatgg
ctttggatggttacctgagatggttcccaaaaagcaaccagagagatgtttgcccaaaggctttggttgctgctttccatgctg
tagcgtggacaagagaaagcctccctgggtcatttggtggaacctgcggaaaacctgctaccaaatagtgaaacacagct
ggtttgagagctttattatctttgtgattctgctgagcagtggggcactgatatttgaagatgttcaccttgagaaccaacccaa
aatccaagaattactaaattgtactgacattatttttacacatatttttatcctggagatggtactaaaatgggtagccttcggattt
ggaaagtatttcaccagtgcctggtgctgccttgatttcatcattgtgattgtctctgtgaccaccctcattaacttaatggaattg
aagtccttccggactctacgagcactgaggcctcttcgtgcgctgtcccagtttgaaggaatgaaggtggtggtcaatgctct
cataggtgccatacctgccattctgaatgttttgcttgtctgcctcattttctggctcgtatttgtattctgggagtatacttcttttct
ggaaaatttgggaaatgcattaatggaacagactcagttataaattataccatcattacaaataaaagtcaatgtgaaagtggc
aatttctcttggatcaaccagaaagtcaactttgacaatgtgggaaatgcttacctcgctctgctgcaagtggcaacatttaag
ggctggatggatattatatatgcagctgttgattccacagagaaagaacaacagccagagtttgagagcaattcactcggtta
catttacttcgtagtctttatcatctttggctcattcttcactctgaatctcttcattggcgttatcattgacaacttcaaccaacagc
 
agaaaaagttaggtggccaagacatttttatgacagaagaacagaagaaatactataatgcaatgaaaaaattaggatccaa
aaaacctcaaaaacccattccacggcctctgaacaaatgtcaaggtctcgtgttcgacatagtcacaagccagatctttgaca
tcatcatcataagtctcattatcctaaacatgattagcatgatggctgaatcatacaaccaacccaaagccatgaaatccatcct
tgaccatctcaactgggtctttgtggtcatctttacgttagaatgtctcatcaaaatctttgctttgaggcaatactacttcaccaat
ggctggaatttatttgactgtgtggtcgtgcttctttccattgttagtacaatgatttctaccttggaaaatcaggagcacattcctt
tccctccgacgctcttcagaattgtccgcttggctcggattggccgaatcctgaggcttgtccgggctgcacgaggaatcag
gactctcctctttgctctgatgatgtcgcttccttctctgttcaacattggtcttctactctttctgattatgtttatctatgccattctg
ggtatgaactggttttccaaagtgaatccagagtctggaatcgatgacatattcaacttcaagacttttgccagcagcatgctct
gtctcttccagataagcacatcagcaggttgggattccctgctcagccccatgctgcgatcaaaagaatcatgtaactcttcct
cagaaaactgccacctccctggcatagccacatcctactttgtcagttacattatcatctcctttctcattgttgtcaacatgtaca
ttgctgtgattttagagaacttcaatacagccactgaagaaagtgaggaccctttgggtgaagatgactttgacatattttatga
agtgtgggaaaagtttgacccagaagcaacacaatttatcaaatattctgccctttctgactttgctgatgccttgcctgagcct
ttgcgtgtcgcaaagccaaataaatatcaatttctagtaatggacttgcccatggtgagtgaagatcgcctccactgcatggat
attcttttcgccttcaccgctagggtactcggtggctctgatggcctagatagtatgaaagcaatgatggaagagaagttcatg
gaagccaatcctctcaagaagttgtatgaacccatagtcaccaccaccaagagaaaggaagaggaaagaggtgctgctat
tattcaaaaggcctttcgaaagtacatgatgaaggtgaccaagggtgaccaaggtgaccaaaatgacttggaaaacgggc
ctcattcaccactccagactctttgcaatggagacttgtctagctttggggtggccaagggcaaggtccactgtgactga
(SEQ ID NO: 15)
H.s. SCN1B 
Atggggaggctgctggccttagtggtcggcgcggcactggtgtcctcagcctgcgggggctgcgtggaggtggactcg
gagaccgaggccgtgtatgggatgaccttcaaaattctttgcatctcctgcaagcgccgcagcgagaccaacgctgagac
cttcaccgagtggaccttccgccagaagggcactgaggagtttgtcaagatcctgcgctatgagaatgaggtgttgcagct
ggaggaggatgagcgcttcgagggccgcgtggtgtggaatggcagccggggcaccaaagacctgcaggatctgtctat
cttcatcaccaatgtcacctacaaccactcgggcgactacgagtgccacgtctaccgcctgctcttcttcgaaaactacgag
cacaacaccagcgtcgtcaagaagatccacattgaggtagtggacaaagccaacagagacatggcatccatcgtgtctga
gatcatgatgtatgtgctcattgtggtgttgaccatatggctcgtggcagagatgatttactgctacaagaagatcgctgccgc
cacggagactgctgcacaggagaatgcctcggaatacctggccatcacctctgaaagcaaagagaactgcacgggcgtc
caggtggccgaatag (SEQ ID NO: 16)
H.s. SCN2B 
Atgcacagagatgcctggctacctcgccctgccttcagcctcacggggctcagtctcttttctctttggtgccaccaggacg
gagcatggaggtcacagtacctgccaccctcaacgtcctcaatggctctgacgcccgcctgccctgcaccttcaactcctg
ctacacagtgaaccacaaacagttctccctgaactggacttaccaggagtgcaacaactgctctgaggagatgttcctccag
ttccgcatgaagatcattaacctgaagctggagcggtttcaagaccgcgtggagttctcagggaaccccagcaagtacgat
gtgtcggtgatgctgagaaacgtgcagccggaggatgaggggatttacaactgctacatcatgaaccccccctgaccgcca
ccgtggccatggcaagatccatctgcaggtcctcatggaagagccccctgagcgggactccacggtggccgtgattgtgg
gtgcctccgtcgggggcttcctggctgtggtcatcttggtgctgatggtggtcaagtgtgtgaggagaaaaaaagagcaga
agctgagcacagatgacctgaagaccgaggaggagggcaagacggacggtgaaggcaacccggatgatggcgccaa
gtag (SEQ ID NO: 17)
H.s. SCN3B 
Atgcctgccttcaatagattgtttcccctggcttctctcgtgcttatctactgggtcagtgtctgcttccctgtgtgtgtggaagt
gccctcggagacggaggccgtgcagggcaaccccatgaagctgcgctgcatctcctgcatgaagagagaggaggtgga
ggccaccacggtggtggaatggttctacaggcccgagggcggtaaagatttccttatttacgagtatcggaatggccacca
ggaggtggagagcccctttcaggggcgcctgcagtggaatggcagcaaggacctgcaggacgtgtccatcactgtgctc
aacgtcactctgaacgactctggcctctacacctgcaatgtgtcccgggagtttgagtttgaggcgcatcggccctttgtgaa
gacgacgcggctgatcccccctaagagtcaccgaggaggctggagaggacttcacctctgtggtctcagaaatcatgatgt
acatccttctggtcttcctcaccttgtggctgctcatcgagatgatatattgctacagaaaggtctcaaaagccgaagaggca
gcccaagaaaacgcgtctgactaccttgccatcccatctgagaacaaggagaactctgcggtaccagtggaggaatag
(SEQ ID NO: 18)
H.s. SCN4B 
Atgcccggggctggggacggaggcaaagccccggcgagatggctgggcactgggcttttgggcctcttcctgctcccc
gtaaccctgtcgctggaggtgtctgtgggaaaggccaccgacatctacgctgtcaatggcacggagatcctgctgccctgc
accttctccagctgctttggcttcgaggacctccacttccggtggacctacaacagcagtgacgcattcaagattctcataga
ggggactgtgaagaatgagaagtctgaccccaaggtgacgttgaaagacgatgaccgcatcactctggtaggctctacta
aggagaagatgaacaacatttccattgtgctgagggacctggagttcagcgacacgggcaaatacacctgccatgtgaag
aaccccaaggagaataatctccagcaccacgccaccatcttcctccaagtcgttgatagactggaagaagtggacaacac
agtgacactcatcatcctggctgtcgtgggcggggtcatcgggctcctcatcctcatcctgctgatcaagaaactcatcatctt
catcctgaagaagactcgggagaagaagaaggagtgtctcgtgagctcctcggggaatgacaacacggagaacggcttg
cctggctccaaggcagaggagaaaccaccttcaaaagtgtga (SEQ ID NO: 19)
H.s. SCN1A 
meqtvlvppgpdsfnfftreslaaierriaeekaknpkpdkkdddengpkpnsdleagknlpfiygdippemvsepl
edldpyyinkktfivlnkgkaifrfsatsalyiltpfnplrkiaikilvhslfsmlimctiltncvfmtmsnppdwtknvey
tftgiytfeslikiiargfcledftflrdpwnwldftvitfayvtefvdlgnvsalrtfrvlralktisvipglktivgaliqsvkkl
sdvmiltvfclsvfaliglqlfmgnlrnkciqwpptnasleehsieknitvnyngtlinetvfefdwksyiqdsryhyfle
gfldallcgnssdagqcpegymcvkagrnpnygytsfdtfswaflslfrlmtqdfwenlyqltlraagktymiffvlvifl
gsfylinlilavvamayeeqnqatleeaeqkeaefqqmieqlkkqqeaaqqaatatasehsrepsaagrlsdssseaskl
ssksakerrnrrkkrkqkeqsggeekdedefqksesedsirrkgfrfsiegnrltyekryssphqsllsirgslfsprrnsrts
lfsfrgrakdvgsendfaddehstfednesrrdslfvprrhgerrnsnlsqtsrssrmlavfpangkmhstvdcngvvsl
vggpsvptspvgqllpegtttetemrkrrsssfhvsmdfledpsqrqramsiasiltntveeleesrqkcppcwykfsni
fliwdcspywlkvkhvvnlvvmdpfvdlaiticivlntlfmamehypmtdhfnnvltvgnlvftgiftaemflkiiam
dpyyyfqegwnifdgfivtlslvelglanveglsvlrsfrllrvfklakswptlnmlikiignsvgalgnltlvlaiivfifav
vgmqlfgksykdcvckiasdcqlprwhmndffhsflivfrvlcgewietmwdcmevagqamcltvfmmvmvig
nlvvlnlflalllssfsadnlaatdddnemnnlqiavdrmhkgvayvkrkiyefiqqsfirkqkildeikplddlnnkkds
cmsnhtaeigkdldylkdvngttsgigtgssvekyiidesdymsfinnpsltvtvpiavgesdfenlntedfssesdlees
keklnesssssegstvdigapveeqpvvepeetlepeacftegcvqrfkccqinveegrgkqwwnlrrtcfrivehnwf
etfivfmillssgalafediyidqrktiktmleyadkvftyifilemllkwvaygyqtyftnawcwldflivdvslvsltan
algyselgaikslrtlralrplralsrfegmrvvvnallgaipsimnvllvclifwlifsimgvnlfagkfyhcintttgdrfdi
edvnnhtdclkliernetarwknvkvnfdnvgfgylsllqvatfkgwmdimyaavdsrnvelqpkyeeslymylyf
vifiifgsfftlnlfigviidnfnqqkkkfggqdifmteeqkkyynamkklgskkpqkpiprpgnkfqgmvfdfvtrq
vfdisimiliclnmvtmmvetddqseyvttilsrinlvfivlftgecvlklislrhyyftigwnifdfvvvilsivgmflaeli
ekyfvsptlfrvirlarigrilrlikgakgirtllfalmmslpalfniglllflvmfiyaifgmsnfayvkrevgiddmfnfetf
gnsmiclfqittsagwdgllapilnskppdcdpnkvnpgssvkgdcgnpsvgifffvsyiiisflvvvnmyiavilenfs
 
vateesaeplseddfemfyevwekfdpdatqfmefeklsqfaaaleppInlpqpnklqliamdlpmvsgdrihcldil
faftkrvlgesgemdalriqmeerfmasnpskvsyqpitttlkrkqeevsaviiqrayrrhllkrtvkqasftynknkikg
ganllikedmiidrinensitektdltmstaacppsydrvtkpivekheqegkdekakgk (SEQ ID NO: 20)
H.s. SCN2A 
maqsvlvppgpdsfrfftreslaaieqriaeekakrpkqerkdeddengpkpnsdleagkslpfiygdippemvsvpl
edldpyyinkktfivlnkgkaisrfsatpalyiltpfnpirklaikilvhslfnmlimctiltncvfmtmsnppdwtknve
ytftgiytfeslikilargfcledftflrdpwnwldftvitfayvtefvdlgnvsalrtfrvlralktisvipglktivgaliqsvk
klsdvmiltvfclsvfaliglqlfmgnlrnkclqwppdnssfeinitsffnnsldgngttfnrtvsifnwdeyiedkshfyfl
egqndallcgnssdagqcpegyicvkagrnpnygytsfdtfswaflslfrlmtqdfwenlyqltlraagktymiffvlvi
flgsfylinlilavvamayeeqnqatleeaeqkeaefqqmleqlkkqqeeaqaaaaasaesrdfsgaggigvfsesss
vasklssksekelknrrkkkqkeqsgeeekndrvrksesedsirrkgfrfslegsrltyekrfssphqsllsirgslfsprr
nsraslfsfrgrakdigsendfaddehstfedndsrrdslfvphrhgerrhsnvsqasrasrvlpilpmngkmhsavdcn
gvvslvggpstltsagqllpegttteteirkrrsssyhvsmdlledptsrqramsiasiltntmeeleesrqkcppcwykfa
nmcliwdcckpwlkvkhlvnlvvmdpfvdlaiticivlntlfmamehypmteqfssvlsvgnlvftgiftaemflkii
amdpyyyfqegwnifdgfivslslmelglanveglsvlrsfrllrvfklakswptlnmlikiignsvgalgnltlvlaiivfi
favvgmqlfgksykecvckisndcelprwhmhdffhsflivfrvlcgewietmwdcmevagqtmcltvfmnvm
vignlvvlnlflalllssfssdnlaatdddnemnnlqiavgrmqkgidfvkrkirefiqkafvrkqkaldeikpledlnnk
kdscisnhttieigkdlnylkdgngttsgigssvekyvvdesdymsfinnpsltvtvpiavgesdfenlnteefssesdm
eeskeklnatsssegstvdigapaegeqpevepeeslepeacftedcvrkfkccqisieegkgklwwnlrktcykiveh
nwfetfivfmillssgalafediyieqrktiktmleyadkvftyifilemllkwvaygfqvyftnawcwldflivdvslvs
ltanalgyselgaikslrtlralrplralsrfegmrvvvnallgaipsimnvllvclifwlifsimgvnlfagkfyhcinyttg
emfdvsvvnnyseckaliesnqtarwknvkvnfdnvglgylsllqvatfkgwmdimyaavdsrnvelqpkyednl
ymylyfvifiifgsfftlnlfigviidnfnqqkkkfggqdifmteeqkkyynamkklgskkpqkpiprpankfqgmvf
dfvtkqvfdisimiliclnmvtmmvetddqsqemtnilywinlvfivlftgecvlklislryyyftigwnifdfvvvilsi
vgmflaeliekyfvsptlfrvirlarigrilrlikgakgirtllfalmmslpalfniglllflvmfiyaifgmsnfayvkrevgi
ddmfnfetfgnsmiclfqittsagwdgllapilnsgppdcdpdkdhpgssvkgdcgnpsvgifffvsyiiisflvvvn
myiavilenfsvateesaeplseddfemfyevwekfdpdatqfiefaklsdfadaldppllirakpnkvqliamdlpmv
sgdrihcldilfaftkrvlgesgemdalriqmeerfmasnpskvsyepitttlkrkqeevsaiiiqrayrryllkqkvkkvs
siykkdkgkecdgtpikedtlidklnenstpektdmtpsttsppsydsvtkpekekfekdksekedkgkdireskk
(SEQ ID NO: 21)
H.s. SCN3A 
maqallvppgpesfrlftreslaaiekraaeekakkpkkeqdnddenkpkpnsdleagknlpfiygdippemvseple
dldpyyinkktfivmnkgkaifrfsatsalyiltplnpvrkiaikilvhslfsmlimctiltncvfmtlsnppdwtknveyt
ftgiytfeslikilargfcledftflrdpwnwldfsvivmayvtefvdlgnvsalrtfrvlralktisvipglktivgaliqsvk
klsdvmiltvfclsvfaliglqlfmgnlrnkclqwppsdsafetnttsyfngtmdsngtfvnvtmstfnwkdyigddsh
fyvldgqkdpllcgngsdagqcpegyicvkagrnpnygytsfdtfswaflslfrlmtqdywenlyqltlraagktymif
fvlviflgsfylvnlilavvamayeeqnqatleeaeqkeaefqqmleqlkkqqeeaqavaaasaasrdfsgigglgelle
ssseasklssksakewrnrrkkrrqrehlegnnkgerdsfpksesedsvkrssflfsmdgnrltsdkkfcsphqsllsirg
slfsprrnsktsifsfrgrakdvgsendfaddehstfedsesrrdslfvphrhgerrnsnvsqasmssrmvpglpangk
mhstvdcngvvslvggpsaltsptgqlppegtttetevrkrrlssyqismemledssgrqravsiasiltntmeeleesrq
kcppcwyrfanvfliwdccdawlkvkhlvnlivmdpfvdlaiticivlntlfmamehypmteqfssvltvgnlvftgi
ftaemvlkiiamdpyyyfqegwnifdgiivslslmelglsnveglsvlrsfrllrvfklakswptlnmlikiignsvgalg
 
nltlvlaiivfifavvgmqlfgksykecvckinddctlprwhmndffhsflivfrvlcgewietmwdcmevagqtmcl
ivfmlvmvignlvvlnlflalllssfssdnlaatdddnemnnlqiavgrmqkgidyvknkmrecfqkaffrkpkviei
hegnkidscmsnntgieiskelnylrdgngttsgvgtgssvekyvidendymsfinnpsltvtvpiavgesdfenlnte
efssesel eeskeklnatsssegstvdvvlpregeqaetepeedlkpeacftegcikkfpfcqvsteegkgkiwwnlrkt
cysivehnwfetfivfmillssgalafediyieqrktiktmleyadkvftyifilemllkwvaygfqtyftnawcwldfli
vdvslvslvanalgyselgaikslrtlralrplralsrfegmrvvvnalvgaipsimnvllvclifwlifsimgvnlfagkfy
hcvnmttgnmfdisdvnnlsdcqalgkqarwknvkvnfdnvgagylallqvatfkgwmdimyaavdsrdvklqp
vyeenlymylyfvifiifgsfftlnlfigviidnfnqqkkkfggqdifmteeqkkyynamkklgskkpqkpiprpankf
qgmvfdfvtrqvfdisimiliclnmvtmmvetddqgkymtlvlsrinlvfivlftgefvlklvslrhyyftigwnifdfv
vvilsivgmflaemiekyfvsptlfrvirlarigrilrlikgakgirtllfalmmslpalfniglllflvmfiyaifgmsnfayv
kkeagiddmfnfetfgnsmiclfqittsagwdgllapilnsappdcdpdtihpgssvkgdcgnpsvgifffvsyiiisflv
vvnmyiavilenfsvateesaeplseddfemfyevwekfdpdatqfiefsklsdfaaaldpplliakpnkvqliamdlp
mvsgdrihcldilfaftkrvlgesgemdalriqmedrfmasnpskvsyepitttlkrkqeevsaaiiqrnfrcyllkqrlk
nissnynkeaikgridlpikqdmiidklngnstpektdgssstsppsydsvtkpdkekfekdkpekeskgkevrenq
k (SEQ ID NO: 22)
H.s. SCN4A 
marpslctlvplgpeclrpftreslaaieqraveeearlqrnkqmeieeperkprsdleagknlpmiygdpppevigipl
edldpyysnkktfivlnkgkaifrfsatpalyllspfsvvrrgaikvlihalfsmfimitiltncvfmtmsdpppwsknve
ytftgiytfeslikilargfcvddftflrdpwnwldfsvimmayltefvdlgnisalrtfrvlralktitvipglktivgaliqsv
kklsdvmiltvfclsvfalvglqlfmgnlrqkcvrwpppfndtnttwysndtwygndtwygnemwygndswyan
dtwnshaswatndtfdwdayisdegnfyflegsndallcgnssdaghcpegyeciktgrnpnygytsydtfswaflal
frlmtqdywenlfqltlraagktymiffvviiflgsfylinlilavvamayaeqneatlaedkekeeefqqmlekfkkhq
eelekakaaqaleggeadgdpahgkdcngsldtsqgekgaprqsssgdsgisdameeleeahqkcppwwykcah
kvliwnccapwlkfkniihlivmdpfvdlgiticivlntlfmamehypmtehfdnvltvgnlvftgiftaemvlkliam
dpyeyfqqgwnifdsiivtlslvelglanvqglsvlrsfrllrvfklakswptlnmlikiignsvgalgnltlvlaiivfifav
vgmqlfgksykecvckialdcnlprwhmhdffhsflivfrilcgewietmwdcmevagqamcltvflmvmvignl
vvlnlflalllssfsadslaasdedgemnnlqiaigriklgigfakafllgllhgkilspkdimlslgeadgageageageta
pedekkeppeedlkkdnhilnhmgladgppssleldhlnfinnpyltiqvpiaseesdlempteeetdtfsepedskkp
pqplydgnssvcstadykppeedpeeqaeenpegeqpeecfteacvqrwpclyvdisqgrgkkwwtlrracfkive
hnwfetfivfmillssgalafediyieqrrvirtileyadkvftyifimemllkwvaygfkvyftnawcwldflivdvsiis
lvanwlgyselgpikslrtlralrplralsrfegmrvvvnallgaipsimnvllvclifwlifsimgvnlfagkfyycinttts
erfdisevnnksecesImhtgqvrwlnvkvnydnvglgylsllqvatfkgwmdimyaavdsrekeeqpqyevnly
mylyfvifiifgsfftlnlfigviidnfnqqkkklggkdifmteeqkkyynamkklgskkpqkpiprpqnkiqgmvy
dlvtkqafditimiliclnmvtmmvetdnqsqlkvdilyninmifiiiftgecvlkmlalrqyyftvgwnifdfvvvilsi
vglalsdliqkyfvsptlfrvirlarigrvlrlirgakgirtllfalmmslpalfnigllllflvmfiysifgmsnfayvkkesgid
dmfnfetfgnsiiclfeittsagwdgllnpilnsgppdcdpnlenpgtsvkgdcgnpsigicffcsyiiisflivvnmyiai
ilenfnvateesseplgeddfemfyetwekfdpdatqfiaysrlsdfvdtlqeplriakpnkiklitldlpmvpgdkihcl
dilfaltkevlgdsgemdalkqtmeekfmaanpskvsyepitttlkrkheevcaikiqrayrrhllqrsmkqasymyrh
shdgsgddapekegllantmskmyghengnssspspeekgeagdagptmglmpispsdtawppapppgqtvrp
gvkeslv (SEQ ID NO: 23)
H.s. SCN5A 
manflprgtssfrrftreslaaiekrmaekqargsttlqesreglpeeeaprpqldlqaskklpdlygnppqeligepledl
dpfystqktfivlnkgktifrfsatnalyvlspfhpirraavkilvhslfnmlimctiltncvfmaqhdpppwtkyveytft
aiytfeslvkilargfclhaftflrdpwnwldfsviimayttefvdlgnvsalrtfrvlralktisvisglktivgaliqsvkkla
 
dvmvltvfclsvfaliglqlfmgnlrhkcvrnftalngtngsveadglvwesldlylsdpenyllkngtsdvllcgnssda
gtcpegyrclkagenpdhgytsfdsfawaflalfrlmtqdcwerlyqqtlrsagkiymiffmlviflgsfylvnlilavva
mayeeqnqatiaeteekekrfqeamemlkkehealtirgvdtvsrsslemsplapvnsherrskrrkrmssgteecge
drlpksdsedgpramnhlsltrglsrtsmkprssrgsiftfrrrdlgseadfaddenstageseshhtsllvpwplrrtsaqg
qpspgtsapghalhgkknstvdcngvvsllgagdpeatspgshllrpvmlehppdtttpseepggpqmltsqapcvd
gfeepgarqralsavsvltsaleeleesrhkcppcwnrlaqryliweccplwmsikqgvklvvmdpftdltitmcivlnt
lfmalehynmtsefeemlqvgnlvftgiftaemtfkiialdpyyyfqqgwnifdsiivilslmelglsrmsnlsvlrsfrll
rvfklakswptlntlikiignsvgalgnltlvlaiivfifavvgmqlfgknyselrdsdsgllprwhmmdffhafliifrilc
gewietmwdcmevsgqslcllvfllvmvignlvvlnlflalllssfsadnltapdedremnnlqlalariqrglrfvkrtt
wdfccgllrqrpqkpaalaaqgqlpsciatpysppppetekvpptrketrfeegeqpgqgtpgdpepvcvpiavaesd
tddqeedeenslgteeesskqqesqpvsggpeappdsrtwsqvsatasseaeasasqadwrqqwkaepqapgcget
pedscsegstadmtntaelleqipdlgqdvkdpedcftegcvrrcpccavdttqapgkvwwrlrktcyhivehswfet
fiifmillssgalafediyleerktikvlleyadkmftyvfvlemllkwvaygfkkyftnawcwldflivdvslvslvantl
gfaemgpikslrtlralrplralsrfegmrvvvnalvgaipsimnvllvclifwlifsimgvnlfagkfgrcinqtegdlpl
nytivnnksqceslnltgelywtkvkvnfdnvgagylallqvatfkgwmdimyaavdsrgyeeqpqweynlymyi
yfvifiifgsfftlnlfigviidnfnqqkkklggqdifmteeqkkyynamkklgskkpqkpiprplnkyqgfifdivtkq
afdvtimflicInmvtmmvetddqspekinilakinllfvaiftgecivklaalrhyyftnswnifdfvvvilsivgtvlsd
iiqkyffsptlfrvirlarigrilrlirgakgirtllfalmmslpalfnigllllflvmfiysifgmanfayvkweagiddmfnfq
tfansmlclfqittsagwdgllspilntgppycdptlpnsngsrgdcgspavgilffttyiiisflivvnmyiaiilenfsvat
eesteplseddfdmfyeiwekfdpeatqfieysvlsdfadalseplriakpnqislinmdlpmvsgdrihcmdilfaftk
rvlgesgemdalkiqmeekfmaanpskisyepitttlrrkheevsamviqrafrrhllqrslkhasflfrqqagsglseed
aperegliayvmsenfsrplgppssssisstsfppsydsvtratsdnlqvrgsdyshsedladfppspdrdresiv
(SEQ ID NO: 24)
H.s. SCN7A (occasionally referred to as SCN6A) 
mlaspepkglvpftkesfelikqhiakthnedheeedlkptpdlevgkklpfiygnlsqgmvsepledvdpyyykkk
ntfivlnknrtifrfnaasilctlspfncirrttikvlvhpffqlfilisvlidcvfmsltnlpkwrpvlentllgiytfeilvklfar
gvwagsfsflgdpwnwldfsvtvfeviiryspldfiptlqtartlrilkiiplnqglkslvgvlihclkqligviiltlfflsifsli
gmglfmgnlkhkcfrwpqenenetlhnrtgnpyyiretenfyylegeryallcgnrtdagqcpegyvcvkaginpdq
gftnfdsfgwalfalfrlmaqdypevlyhqilyasgkvymiffvvvsflfsfymaslflgilamayeeekqrvgeiskki
epkfqqtgkelqegnetdeaktiqiemkkrspistdtsldvledatlrhkeelekskkicplywykfaktfliwncspcw
lklkefvhriimapftdlfliiciilnvcfltlehypmskqtntllnignlvfigiftaemifkiiamhpygyfqvgwnifds
mivfhglielclanvagmallrlfrmlrifklgkywptfqilmwslsnswvalkdlvlllftfiffsaafgmklfgknyeef
vchidkdcqlprwhmhdffhsflnvfrilcgewvetlwdcmevagqswcipfylmvilignllvlylflalvssfssck
dvtaeenneaknlqlavarikkginyvllkilcktqnvpkdtmdhvnevyvkedisdhtlselsntqdflkdkekssgt
eknatenesqslipspsvsetvpiasgesdienldnkeiqsksgdggskekikqssssecstvdiaiseeeemfyggers
khlkngcrrgsslgqisgaskkgkiwqnirktcckivennwfkcfiglvtllstgtlafediymdqrktikilleyadmift
yifilemllkwmaygfkayfsngwyrldfvvvivfclsligktreelkplismkflrplrvlsqfermkvvvraliktlpt
lnvflvclmiwlifsimgvdlfagrfyecidptsgerfpssevmnksrcesllfnesmlwenakmnfdnvgngflsllq
vatfngwitimnsaidsvavniqphfevniymycyfinfiifgvflplsmlitviidnfnkhkiklggsnifitvkqrkqy
rrlkklmyedsqrpvprplnklqgfifdvvtsqafnvivmvlicfqaiammidtdvqslqmsialywinsifvmlytm
ecilkliafrcfyftiawnifdfmvvifsitglclpmtvgsylvppslvqlillsriihmlrlgkgpkvfhnlmlplmlslpal
lniilliflvmfiyavfgmynfayvkkeagindvsnfetfgnsmlclfqvaifagwdgmldaifnskwsdcdpdkinp
gtqvrgdcgnpsvgifyfvsyilisw liivnmyivvvmeflniaskkknktlseddfrkffqvwkrfdpdrtqyidsskl
sdfaaaldpplfmakpnkgqlialdlpmavgdrihcldillaftkrvmgqdvrmekvvseiesgfllanpfkitcepittt
lkrkqeavsatiiqrayknyrlrrndkntsdihmidgdrdvhatkegayfdkakekspiqsqi (SEQ ID
NO: 25)
H.s. SCN8A 
maarllappgpdsfkpftpeslanierriaesklkkppkadgshreddedskpkpnsdleagkslpfiygdipqglvav
pledfdpyyltqktfvvlnrgktlfrfsatpalyilspfnlirriaikilihsvfsmiimctiltncvfmtfsnppdwsknvey
tftgiytfeslvkiiargfcidgftflrdpwnwldfsvimmayitefvnlgnvsalrtfrvlralktisvipglktivgaliqsv
kklsdvmiltvfclsvfaliglqlfmgnlrnkcvvwpinfnesylengtkgfdweeyinnktnfytvpgmlepllcgns
sdagqcpegyqcmkagrnpnygytsfdtfswaflalfrlmtqdywenlyqltlraagktymiffvlvifvgsfylvnlil
avvamayeeqnqatleeaeqkeaefkamleqlkkqqeeaqaaamatsagtvsedaieeegeegggsprsseiskls
sksakerrnrrkkrkqkelsegeekgdpekvfksesedgmrrkafrlpdnrigrkfsimnqsllsipgspflsrhnskssi
fsfrgpgrfrdpgsenefaddehstveesegrrdslfipirarerrssysgysgysqgsrssrifpslrrsvkrnstvdcngv
vsliggpgshiggrllpeatteveikkkgpgsllvsmdqlasygrkdrinsimsvvtntlveeleesqrkcppcwykfan
tfliwechpywiklkeivnlivmdpfvdlaiticivlntlfmamehhpmtpqfehvlavgnlvftgiftaemflkliam
dpyyyfqegwnifdgfivslslmelsladveglsvlrsfrllrvfklakswptlnmlikiignsvgalgnltlvlaiivfifav
vgmqlfgksykecvckinqdcelprwhmhdffhsflivfrvlcgewietmwdcmevagqamclivfmmvmvi
gnlvvlnlflalllssfsadnlaatdddgemnnlqisvirikkgvawtklkvhafmqahfkqreadevkpldelyekka
ncianhtgadihrngdfqkngngttsgigssvekyiidedhmsfinnpnltvrvpiavgesdfenlntedvssesdpeg
skdklddtsssegstidikpeveevpveqpeeyldpdacftegcvqrfkccqvnieeglgkswwilrktcflivehnwf
etfiifmillssgalafediyieqrktirtileyadkvftyifilemllkwtaygfvkfftnawcwldflivavslvslianalg
yselgaikslrtlralrplralsrfegmrvvvnalvgaipsimnvllvclifwlifsimgvnlfagkyhycfnetseirfeied
vnnkteceklmegnnteirwknvkinfdnvgagylallqvatfkgwmdimyaavdsrkpdeqpkyedniymyiy
fvifiifgsfftlnlfigviidnfnqqkkkfggqdifmteeqkkyynamkklgskkpqkpiprplnkiqgivfdfvtqqa
fdivimmliclnmvtmmvetdtqskqmenilywinlvfvifftcecvlkmfalrhyyftigwnifdfvvvilsivgmf
ladiiekyfvsptlfrvirlarigrilrlikgakgirtllfalmmslpalfnigllllflvmfifsifgmsnfayvkheagiddmf
nfetfgnsmiclfqittsagwdglllpilnrppdcsldkehpgsgfkgdcgnpsvgifffvsyiiisflivvnmyiaiilenf
svateesadplseddfetfyeiwekfdpdatqfieyckladfadalehplrvpkpntieliamdlpmvsgdrihcldilfa
ftkrvlgdsgeldilrqqmeerfvasnpskvsyepitttlrrkqeevsavvlqrayrghlarrgfickkttsnklenggthre
kkestpstaslpsydsvtkpekekqqraeegrrerakrqkevreskc (SEQ ID NO: 26)
H.s. SCN9A 
mamlpppgpqsfvhftkqslalieqriaerkskepkeekkdddeeapkpssdleagkqlpfiygdippgmvsepled
ldpyyadkktfivlnkgktifrfnatpalymlspfsplrrisikilvhslfsmlimctiltncifmtmnnppdwtknveytf
tgiytfeslvkilargfcvgeftflrdpwnwldfvvivfayltefvnlgnvsalrtfrvlralktisvipglktivgaliqsvkkl
sdvmiltvfclsvfaliglqlfmgnlkhkcfrnslennetlesimntleseedfrkyfyylegskdallcgfstdsgqcpeg
ytcvkigrnpdygytsfdtfswaflalfrlmtqdywenlyqqtlraagktymiffvvviflgsfylinlilavvamayeeq
nqanieeakqkelefqqmldrlkkeqeeaeaiaaaaaeytsirrsrimglsesssetsklssksakerrnrrkkknqkkls
sgeekgdaeklsksesedsirrksfhlgveghrrahekrlstpnqsplsirgslfsarrssrtslfsfkgrgrdigsetefadde
hsifgdnesrrgslfvphrpqerrssnisqasrsppmlpvngkmhsavdcngvvslvdgrsalmlpngqllpegttnqi
hkkrrcssyllsedmlndpnlrqramsrasiltntveeleesrqkcppwwyrfahkfliwncspywikfkkciyfivm
dpfvdlaiticivlntlfmamehhpmteefknvlaignlvftgifaaemvlkliamdpyeyfqvgwnifdslivtlslve
lfladveglsvlrsfrllrvfklakswptlnmlikiignsvgalgnltlvlaiivfifavvgmqlfgksykecvckinddctlp
rwhmndffhsflivfrvlcgewietmwdcmevagqamclivymmvmvignlvvlnlflalllssfssdnltaieedp
dannlqiavtrikkginyvkqtlrefilkafskkpkisreirqaedlntkkenyisnhtlaemskghnflkekdkisgfgss
vdkhlmedsdgqsfihnpsltvtvpiapgesdlenmnaeelssdsdseyskvrlnrssssecstvdnplpgegeeaeae
pmnsdepeacftdgcvrrfsccqvniesgkgkiwwnirktcykivehswfesfivlmillssgalafediyierkktikii
leyadkiftyifilemllkwiaygyktyftnawcwldflivdvslvtlvantlgysdlgpikslrtlralrplralsrfegmrv
vvnaligaipsimnvllvclifwlifsimgvnlfagkfyecinttdgsrfpasqvpnrsecfalmnvsqnvrwknlkvnf
dnvglgylsllqvatfkgwtiimyaavdsvnvdkqpkyeyslymyiyfvvfiifgsfftlnlfigviidnfnqqkkklgg
 
qdifmteeqkkyynamkklgskkpqkpiprpgnkiqgcifdlvtnqafdisimvliclnmvtmmvekegqsqhm
tevlywinvvfiilftgecvlklislrhyyftvgwnifdfvvviisivgmfladlietyfvsptlfrvirlarigrilrlvkgakg
irtllfalmmslpalfniglllflvmfiyaifgmsnfayvkkedgindmfnfetfgnsmiclfqittsagwdgllapilnsk
ppdcdpkkvhpgssvegdcgnpsvgifyfvsyiiisflvvvnmyiavilenfsvateesteplseddfemfyevwekf
dpdatqfiefsklsdfaaaldppllliakpnkvqliamdlpmvsgdrihcldilfaftkrvlgesgemdslrsqmeerfms
anpskvsyepitttlkrkqedvsatviqrayrryrlrqnvknissiyikdgdrdddllnkkdmafdnvnensspektdat
ssttsppsydsvtkpdkekyeqdrtekedkgkdskeskk (SEQ ID NO: 27)
H.s. SCN10A 
mefpigsletnnfrrftpeslveiekqiaakqgtkkarekhreqkdqeekprpqldlkacnqlpkfygelpaeligeple
dldpfysthrtfmvlnkgrtisrfsatralwlfspfnlirrtaikvsvhswfslfitvtilvncvcmtrtdlpekieyvftviytf
ealikilargfclneftylrdpwnwldfsvitlayvgtaidlrgisglrtfrvlralktvsvipglkvivgalihsvkkladvtilt
ifclsvfalvglqlfkgnlknkcvkndmavnettnysshrkpdiyinkrgtsdpllcgngsdsghcpdgyiclktsdnp
dfnytsfdsfawaflslfrlmtqdswerlyqqtlrtsgkiymiffvlviflgsfylvnlilavvtmayeeqnqattdeieake
kkfqealemlrkeqevlaalgidttslhshngspltsknaserrhrikprvsegstednksprsdpynqrrmsflglasgk
rrashgsvfhfrspgrdislpegvtddgvfpgdheshrgslllgggagqqgplprsplpqpsnpdsrhgedehqpppts
elapgavdvsafdagqkktflsaeyldepfraqramsvvsiitsvleeleeseqkcppcltslsqkyliwdccpmwvkl
ktilfglvtdpfaeltitlcivvntifmamehhgmsptfeamlqignivftifftaemvfkiiafdpyyyfqkkwnifdcii
vtvsllelgvakkgslsvlrsfrllrvfklakswptlntlikiignsvgalgnltiilaiivfvfalvgkqllgenyrnnrknisa
phedwprwhmhdffhsflivfrilcgewienmwacmevgqksiclilfltvmvlgnlvvlnlfialllnsfsadnltap
eddgevnnlqvalariqvfghrtkqalcsffsrscpfpqpkaepelvvklplsssskaenhiaantargssgglqaprgprd
ehsdfianptvwvsvpiaegesdlddleddggedaqsfqqevipkgqqeqlqqvercgdhltprspgtgtssedlapsl
getwkdesvpqvpaegvddtsssegstvdcldpeeilrkipeladdleepddcftegcirhcpcckldttkspwdvgw
qvrktcyrivehswfesfiifmillssgslafedyyldqkptvkalleytdrvftfifvfemllkwvaygfkkyftnawcw
ldflivnislisltakileysevapikalrtlralrplralsrfegmrvvvdalvgaipsimnvllvclifwlifsimgvnlfag
kfwrcinytdgefslvplsivnnksdckiqnstgsffwvnvkvnfdnvamgylallqvatfkgwmdimyaavdsre
vnmqpkwednvymylyfvifiifggfftlnlfvgviidnfnqqkkklggqdifmteeqkkyynamkklgskkpqk
piprplnkfqgfvfdivtrqafditimvliclnmitmmvetddqseektkilgkinqffvavftgecvmkmfalrqyyft
ngwnvfdfivvvlsiaslifsailkslqsyfsptlfrvirlarigrilrliraakgirtllfalmmslpalfnigllllflvmfiysifg
mssfphvrweagiddmfnfqtfansmlclfqittsagwdgllspilntgppycdpnlpnsngtrgdcgspavgiifftty
iiisflimvnmyiavilenfnvateesteplseddfdmfyetwekfdpeatqfitfsalsdfadtlsgplripkpnrniliq
mdlplvpgdkihcldilfaftknvlgesgeldslkanmeekfmatnlskssyepiattlrwkqedisatviqkayrsyvl
hrsmalsntpcvpraeeeaaslpdegfvaftanencvlpdksetasatsfppsyesvtrglsdrvnmrtsssiqnedeats
meliapgp (SEQ ID NO: 28)
H.s. SCN11A 
mddrcypvifpdernfrpftsdslaaiekriaiqkekkkskdqtgevpqprpqldlkasrklpklygdipreligkpledl
dpfyrnhktfmvlnrkrtiyrfsakhalfifgpfnsirslairvsvhslfsmfiigtviincvfmatgpaknsnsnntdiaec
vftgiyifealikilargfildefsflrdpwnwldsivigiaivsyipgitikllplrtfrvfralkaisvvsrlkvivgallrsvkk
lvnviiltffclsifalvgqqlfmgslnlkcisrdcknisnpeaydhcfekkenspefkmcgiwmgnsacsiqyeckht
kinpdynytnfdnfgwsflamfrlmtqdsweklyqqtlrttglysvfffivviflgsfylinltlavvtmayeeqnknvaa
eieakekmfqeaqqllkeekealvamgidrssltsletsyftpkkrklfgnkkrksfflresgkdqppgsdsdedcqkk
pqlleqtkrlsqnlsldhfdehgdplqrqralsavsiltitmkeqeksqepclpcgenlaskylvwnccpqwlcvkkvlr
tvmtdpftelaiticiiintvflamehhkmeasfekmlnignlvftsifiaemclkiialdpyhyfrrgwnifdsivallsfa
dvmncvlqkrswpflrsfrvlrvfklakswptlntlikiignsvgalgsltvvlvivififsvvgmqlfgrsfnsqkspklc
nptgptvsclrhwhmgdfwhsflvvfrilcgewienmwecmqeanassslcvivfilitvigklvvlnlfialllnsfsn
eerngnlegearktkvqlaldrfrrafcfvrhtlehfchkwcrkqnlpqqkevaggcaaqskdiiplvmemkrgsetqe
elgiltsvpktlgvrhdwtwlaplaeeeddvefsgednaqritqpepeqqayelhqenkkptsqrvqsveidmfsede
phltiqdprkksdvtsilsecstidlqdgfgwlpemvpkkqperclpkgfgccfpccsvdkrkppwviwwnlrktcy
qivkhswfesfiifvillssgalifedvhlenqpkiqellnctdiifthifilemvlkwvafgfgkyftsawccldfiivivsv
ttlinlmelksfrtlralrplralsqfegmkvvvnaligaipailnvllvclifwlvfcilgvyffsgkfgkcingtdsvinytii
tnksqcesgnfswinqkvnfdnvgnaylallqvatfkgwmdiiyaavdstekeqqpefesnslgyiyfvvfiifgsfftl
nlfigviidnfnqqqkklggqdifmteeqkkyynamkklgskkpqkpiprplnkcqglvfdivtsqifdiiiisliilnm
ismmaesynqpkamksildhlnwvfvviftleclikifalrqyyftngwnlfdcvvvllsivstmistlenqehipfpptl
frivrlarigrilrlvraargirtllfalmmslpslfnigllllflimfiyailgmnwfskvnpesgiddifnfktfassmlclfqi
stsagwdsllspmlrskescnsssenchlpgiatsyfvsyiiisflivvnmyiavilenfntateesedplgeddfdifyev
wekfdpeatqfikysalsdfadalpeplrvakpnkyqflvmdlpmvsedrlhcmdilfaftarvlggsdgldsmkam
meekfmeanplkklyepivtttkrkeeergaaiiqkafrkymmkvtkgdqgdqndlengphsplqtlcngdlssfgv
akgkvhcd (SEQ ID NO: 29)
H.s. SCN1B 


 (SEQ ID NO: 30)
H.s. SCN2B 
Mhrdawlprpafsltglslffslvppgrsmevtvpatlnvlngsdarlpctfnscytvnhkqfslnwtyqecnncseem
flqfrmkiinlklerfqdrvefsgnpskydvsvmlrnvqpedegiyncyimnppdrhrghgkihlqvlmeepperds
tvavivgasvggflavvilvlmvvkcvrrkkeqklstddlkteeegktdgegnpddgak (SEQ ID NO: 31)
H.s. SCN3B 
Mpafnrlfplaslvliywvsvcfpvcvevpseteavqgnpmklrciscmkreeveattvvewfyrpeggkdfliyey
rnghqevespfqgrlqwngskdlqdvsitvlnvtlndsglytcnvsrefefeahrpfvkttrliplrvteeagedftsvvse
immyillvfltlwlliemiycyrkvskaeeaaqenasdylaipsenkensavpvee (SEQ ID NO: 32)
H.s. SCN4B 
Mpgagdggkaparwlgtgllglfllpvtlslevsvgkatdiyavngteillpctfsscfgfedlhfrwtynssdafkiliegt
vkneksdpkvtlkdddritlvgstkekmnnisivlrdlefsdtgkytchvknpkennlqhhatiflqvvdrleevdntvtl
iilavvggviglliliillikkliifilkktrekkkeclvsssgndntenglpgskaeekppskv (SEQ ID NO: 33)
Signaling probe 3 - (binds target 3)
   **5'- Fam** GCGAGAGCGACAAGCAGACCCTATAGAACCTCGC **BHQ1 quench - 3'** (SEQ ID NO: 34)

## Claims

1. A cell line, or a cell from the cell line, engineered to stably express one heterologous human NaV alpha 9 subunit and two different human NaV beta subunits of a human NaV protein, wherein the human NaV subunits form a human NaV 1.7, wherein the two different human NaV beta subunits are a beta 1 subunit and a beta 2 subunit.

2. The cell or cell line of claim 1, wherein the cell or cell line is grown or maintained in the absence of antibiotics.

3. The cell or cell line of claim 1 or 2, wherein the cell or cell line does not express the human NaV protein endogenously.

4. The cell or cell line of any one of claims 1-3, wherein the heterologous human NaV alpha 9 subunit comprises the amino acid sequence set forth in SEQ ID NO: 27.

5. The cell or cell line of any one of claims 1-3, wherein the heterologous human NaV alpha 9 subunit is encoded by a nucleic acid sequence set forth in SEQ ID NO: 13.

6. The cell or cell line of any one of claims 1-5, wherein each of the two different human NaV beta subunits are individually selected from the group consisting of:
a beta 1 subunit having the amino acid sequence set forth in SEQ ID NO:30;
a beta 2 subunit having the amino acid sequence set forth in SEQ ID NO:31;
a polypeptide with at least 95% sequence identity to any one of SEQ ID NOS: 30-31 that modulates a voltage-gated ion channel;
a polypeptide encoded by any one of the nucleic acid sequences of SEQ ID NOS: 16-17;
a polypeptide encoded by a nucleic acid that hybridizes under stringent conditions to any one of SEQ ID NOS: 16-17; and
a polypeptide encoded by a nucleic acid with at least 95% sequence identity to any one of SEQ ID NOS: 16-17.

7. A method for producing the cell or cell line of any one of claims 1-6, comprising the steps of:
(a) introducing into host cells a coding sequence for a human NaV alpha 9 subunit, a coding sequence for a first human NaV beta subunit and a coding sequence for a second human NaV beta subunit, said coding sequences being linked operably to transcriptional regulatory sequences, wherein the first and second human NaV beta subunits are different, wherein the first and second human NaV beta subunits are a beta 1 subunit and a beta 2 subunit;
(b) introducing into the host cells a first molecular beacon that detects the expression of the human NaV alpha 9 subunit, a second molecular beacon that detects the expression of the first human NaV beta subunit and a third molecular beacon that detects the expression of the second human NaV beta subunit; and
(c) isolating a cell line that stably expresses the human NaV alpha 9 subunit, the first human NaV beta subunit and the second human NaV beta subunit, thereby obtaining the cell or cell line of any one of claims 1-6.

8. The method of claim 7, wherein the host cells are 293T cells.

9. The method of claim 7, wherein the detection is of a fluorescent signal.

10. The method of claim 7, wherein the isolating utilizes a fluorescence activated cell sorter (FACS).

11. A method for identifying a human NaV 1.7 modulator, comprising
contacting a plurality of cells from the cell or cell line of any one of claims 1-6 with a test compound; and
detecting a change in a human NaV 1.7 function in the contacted cells compared to cells not contacted with the test compound, wherein a change in said function indicates that the test compound is a human NaV 1.7 modulator.

12. The method of claim 11, wherein the test compound is a small molecule, a polypeptide, a peptide, or an antibody or an antigen-binding portion thereof.

13. The method of claim 11, wherein the test compound is a library of compounds.

## Patentansprüche

1. Zelllinie, oder Zelle aus der Zelllinie, die gentechnisch verändert ist, um eine heterologe humane NaV-alpha-9-Untereinheit und zwei verschiedene humane NaV-beta-Untereinheiten eines humanen NaV-Proteins stabil zu exprimieren, wobei die humanen NaV-Untereinheiten ein humanes NaV 1.7 bilden, wobei die zwei verschiedenen humanen NaV-beta-Untereinheiten eine Beta-1-Untereinheit und eine Beta-2-Untereinheit sind.

2. Zelle oder Zelllinie nach Anspruch 1, wobei die Zelle oder die Zelllinie in Abwesenheit von Antibiotika gezüchtet oder aufrechterhalten wird.

3. Zelle oder Zelllinie nach Anspruch 1 oder 2, wobei die Zelle oder die Zelllinie das humane NaV-Protein nicht endogen exprimiert.

4. Zelle oder Zelllinie nach einem der Ansprüche 1 bis 3, wobei die heterologe humane NaV-alpha-9-Untereinheit die in SEQ ID NO:27 gezeigte Aminosäuresequenz umfasst.

5. Zelle oder Zelllinie nach einem der Ansprüche 1 bis 3, wobei die heterologe humane NaV-alpha-9-Untereinheit von einer wie in SEQ ID NO:13 gezeigten Nucleinsäuresequenz codiert wird.

6. Zelle oder Zelllinie nach einem der Ansprüche 1 bis 5, wobei jede der beiden verschiedenen humanen NaV-beta-Untereinheiten einzeln ausgewählt sind aus der Gruppe bestehend aus:
einer Beta-1-Untereinheit, die die in SEQ ID NO:30 gezeigte Aminosäuresequenz aufweist;
einer Beta-2-Untereinheit, die die in SEQ ID NO:31 gezeigte Aminosäuresequenz aufweist;
einem Polypeptid mit mindestens 95% Sequenzidentität mit einer der SEQ ID NOs:30 bis 31, das einen spannungsgesteuerten lonenkanal (voltage-gated ion channel) moduliert;
einem Polypeptid, das von einer der Nucleinsäuresequenzen der SEQ ID NOs:16 bis 17 codiert wird;
einem Polypeptid, das von einer Nucleinsäure codiert wird, die unter stringenten Bedingungen an eine der SEQ ID NOs:16 bis 17 hybridisiert; und
einem Polypeptid, das von einer Nucleinsäure mit mindestens 95% Sequenzidentität mit einer der SEQ ID NOs:16 bis 17 codiert wird.

7. Verfahren zur Herstellung der Zelle oder Zelllinie nach einem der Ansprüche 1 bis 6, umfassend die Schritte:
(a) Einbringen in Wirtszellen einer codierenden Sequenz für eine humane NaV-alpha-9-Untereinheit, einer codierenden Sequenz für eine erste humane NaV-beta-Untereinheit und einer codierenden Sequenz für eine zweite humane NaV-beta-Untereinheit, wobei die codierenden Sequenzen mit transkriptionellen regulatorischen Sequenzen funktionell verknüpft sind, wobei sich die erste und die zweite humane NaV-beta-Untereinheit unterscheiden, wobei die erste und die zweite humane NaV-beta-Untereinheiten eine Beta-1-Untereinheit und eine Beta-2-Untereinheit sind;
(b) Einbringen in die Wirtszellen eines ersten molekularen Beacons, das die Expression der humanen NaV-alpha-9-Untereinheit nachweist, eines zweiten molekularen Beacons, das die Expression der ersten humanen NaV-beta-Untereinheit nachweist, und eines dritten molekularen Beacons, das die Expression der zweiten humanen NaV-beta-Untereinheit nachweist; und
(c) Isolieren einer Zelllinie, die die humane NaV-alpha-9-Untereinheit, die erste humane NaV-beta-Untereinheit und die zweite humane NaV-beta-Untereinheit stabil exprimiert, wodurch die Zelle oder die Zelllinie nach einem der Ansprüche 1 bis 6 erhalten wird.

8. Verfahren nach Anspruch 7, wobei die Wirtszellen 293T-Zellen sind.

9. Verfahren nach Anspruch 7, wobei der Nachweis der eines Fluoreszenzsignals ist.

10. Verfahren nach Anspruch 7, wobei die Isolierung einen Fluoreszenz-aktivierten Zellsortierer (FACS) verwendet.

11. Verfahren zum Identifizieren eines humanen NaV 1.7-Modulators, umfassend:
Inkontaktbringen einer Vielzahl von Zellen von der Zelle oder der Zelllinie nach einem der Ansprüche 1 bis 6 mit einer Testverbindung; und
Nachweisen einer Veränderung einer Funktion des humanen NaV 1.7 in den in Kontakt gebrachten Zellen im Vergleich zu Zellen, die nicht mit der Testverbindung in Kontakt gebracht wurden, wobei eine Veränderung in der Funktion darauf hinweist, dass die Testverbindung ein humaner NaV 1.7-Modulator ist.

12. Verfahren nach Anspruch 11, wobei die Testverbindung ein niedermolekulares Molekül, ein Polypeptid, ein Peptid oder ein Antikörper oder ein Antigenbindender Teil davon ist.

13. Verfahren nach Anspruch 11, wobei die Testverbindung eine Bibliothek von Verbindungen ist.

## Revendications

1. Lignée cellulaire, ou cellule issue de la lignée cellulaire, génétiquement modifiée pour exprimer de manière stable une sous-unité alpha 9 hétérologue de NaV humaine et deux sous-unités bêta différentes de NaV humaine d'une protéine NaV humaine, dans laquelle les sous-unités de NaV humaine forment une NaV 1.7 humaine, et les deux sous-unités bêta différentes de NaV humaine sont une sous-unité bêta 1 et une sous-unité bêta 2.

2. Cellule ou lignée cellulaire selon la revendication 1, dans laquelle la cellule ou lignée cellulaire est cultivée ou maintenue en l'absence d'antibiotiques.

3. Cellule ou lignée cellulaire selon la revendication 1 ou 2, dans laquelle la cellule ou lignée cellulaire n'exprime pas la protéine NaV humaine par voie endogène.

4. Cellule ou lignée cellulaire selon l'une quelconque des revendications 1 à 3, dans laquelle la sous-unité alpha 9 hétérologue de NaV humaine comprend la séquence d'acides aminés représentée dans SEQ ID No : 27.

5. Cellule ou lignée cellulaire selon l'une quelconque des revendications 1 à 3, dans laquelle la sous-unité alpha 9 hétérologue de NaV humaine est codée par une séquence d'acide nucléique représentée dans SEQ ID No : 13.

6. Cellule ou lignée cellulaire selon l'une quelconque des revendications 1 à 5, dans laquelle chacune des deux sous-unités bêta différentes de NaV humaine sont individuellement choisies dans le groupe consistant en :
une sous-unité bêta 1 ayant la séquence d'acides aminés représentée dans SEQ ID No : 30 ;
une sous-unité bêta 2 ayant la séquence d'acides aminés représentée dans SEQ ID No : 31 ;
un polypeptide présentant une identité de séquence d'au moins 95 % avec l'une quelconque des SEQ ID No : 30-31 qui module un canal ionique dépendant du potentiel ;
un polypeptide codé par l'une quelconque des séquences d'acide nucléique de SEQ ID No : 16-17 ;
un polypeptide codé par un acide nucléique qui s'hybride dans des conditions stringentes à l'une quelconque des SEQ ID No : 16-17 ; et
un polypeptide codé par un acide nucléique présentant une identité de séquence d'au moins 95 % avec l'une quelconque des SEQ ID No : 16-17.

7. Méthode de production de la cellule ou lignée cellulaire selon l'une quelconque des revendications 1 à 6, comprenant les étapes de :
(a) introduction dans des cellules hôtes d'une séquence codante pour une sous-unité alpha 9 de NaV humaine, d'une séquence codante pour une première sous-unité bêta de NaV humaine et d'une séquence codante pour une seconde sous-unité bêta de NaV humaine, lesdites séquences codantes étant fonctionnellement liées à des séquences régulatrices transcriptionnelles, dans laquelle les première et seconde sous-unités bêta de NaV humaine sont différentes, et les première et seconde sous-unités bêta de NaV humaine sont une sous-unité bêta 1 et une sous-unité bêta 2 ;
(b) introduction dans les cellules hôtes d'une première balise moléculaire qui détecte l'expression de la sous-unité alpha 9 de NaV humaine, d'une deuxième balise moléculaire qui détecte l'expression de la première sous-unité bêta de NaV humaine et d'une troisième balise moléculaire qui détecte l'expression de la seconde sous-unité bêta de NaV humaine ; et
(c) isolement d'une lignée cellulaire qui exprime de manière stable la sous-unité alpha 9 de NaV humaine, la première sous-unité bêta de NaV humaine et la seconde sous-unité bêta de NaV humaine, pour obtenir ainsi la cellule ou lignée cellulaire selon l'une quelconque des revendications 1 à 6.

8. Méthode selon la revendication 7, dans laquelle les cellules hôtes sont des cellules 293T.

9. Méthode selon la revendication 7, dans laquelle la détection est celle d'un signal fluorescent.

10. Méthode selon la revendication 7, dans laquelle l'isolement utilise un trieur cellulaire activé par florescence (FACS).

11. Méthode pour identifier un modulateur de NaV 1.7 humaine, comprenant
la mise en contact d'une pluralité de cellules issues de la cellule ou lignée cellulaire selon l'une quelconque des revendications 1 à 6 avec un composé d'essai ; et
la détection d'un changement dans une fonction de la NaV 1.7 humaine dans les cellules ayant été mises en contact, comparativement aux cellules n'ayant pas été mises en contact, avec le composé d'essai, où le changement dans ladite fonction indique que le composé d'essai est un modulateur de NaV 1.7 humaine.

12. Méthode selon la revendication 11, dans laquelle le composé d'essai est une petite molécule, un polypeptide, un peptide, ou un anticorps ou partie de celui-ci se liant à l'antigène.

13. Méthode selon la revendication 11, dans laquelle le composé d'essai est une banque de composés.
